# EUROPEAN PATENT SPECIFICATION

(11) **EP 2 525 806 B1**
(45) Date of publication and mention of the grant of the patent: **04.06.2014**
(21) Application number: 11700371.5
(22) Date of filing: 19.01.2011
(51) Int. Cl.: A61K 38/18, A61L 27/54, A61L 29/16, A61L 31/16, C12N 15/11, C12Q 1/68, A61P 9/10, A61P 9/14, C07K 14/575, A61K 38/00

(54) **Angiopoietin-like-protein-4 (ANGPTL4) for the preservation of vascular endothelial-cell barrier integrity**
Angiopoietin-ähnliche-Protein-4-(ANGPTL4)-Polypeptide zur Verwendung bei der Erhaltung der Barrierenintegrität vaskulärer Endothelzellen
Polypeptides de protéine 4 de type angiopoïétine (ANGPTL4) pour une utilisation dans la conservation de l'intégrité de la barrière cellulaire de l'endothélium vasculaire

(30) Priority: 20.01.2010 EP 10305063
(43) Date of publication of application: 28.11.2012
(73) Proprietor: INSERM (Institut National de la Santé et de la Recherche Médicale), 75013 Paris (FR)
(72) Inventor: GERMAIN, Stéphane, F-75005 Paris (FR); GALAUP, Ariane, F-75005 Paris (FR); MONNOT, Catherine, F-75005 Paris (FR); TISSIER, Renaud, F-94010 Cedex Creteil (FR); GHALEH, Bijan, F-94010 Cedex Creteil (FR); BERDEAUX, Alain, F-94010 Cedex Creteil (FR)
(74) Representative: Hirsch, Denise Marie
(86) International application number: PCT/EP2011/050682
(87) International publication number: WO 2011/089152

(56) References cited:
- EP-A1- 1 308 511
- WO-A2-2006/014678
- ITO Y ET AL: "Inhibition of angiogenesis and vascular leakiness by angiopoietin-related protein 4", CANCER RESEARCH, AMERICAN ASSOCIATION FOR CANCER RESEARCH, BALTIMORE, MD., US, vol. 63, no. 20, 15 October 2003 (2003-10-15), pages 6651-6657, XP002373803, ISSN: 0008-5472
- ITO Y ET AL: "MOLECULAR CLONING, EXPRESSION AND CHARACTERIZATION OF A NOVEL ANGIOPOIETIN - RELATED PROTEIN, ARP4", ARVO ANNUAL MEETING ABSTRACT SEARCH AND PROGRAM PLANNER,, 4 May 2003 (2003-05-04), XP009131838,
- GALAUP ARIANE ET AL: "Angiopoietin-like 4 prevents metastasis through inhibition of vascular permeability and tumor cell motility and invasiveness", PROCEEDINGS OF THE NATIONAL ACADEMY OF SCIENCES OF THE UNITED STATES OF AMERICA, vol. 103, no. 49, December 2006 (2006-12), pages 18721-18726, XP002576800, ISSN: 0027-8424
- KIM I ET AL: "Hepatic expression, synthesis and secretion of a novel fibrinogen/angiopoietin-related protein that prevents endothelial-cell apoptosis", BIOCHEMICAL JOURNAL, THE BIOCHEMICAL SOCIETY, LONDON, GB LNKD- DOI:10.1042/0264-6021:3460603, vol. 346, no. 3, 15 March 2000 (2000-03-15), pages 603-610, XP002154527, ISSN: 0264-6021
- FOLSOM A R ET AL: "Variation in ANGPTL4 and risk of coronary heart disease: the Atherosclerosis Risk in Communities Study", METABOLISM, CLINICAL AND EXPERIMENTAL, W.B. SAUNDERS CO., PHILADELPHIA, PA, US LNKD- DOI:10.1016/J.METABOL.2008.06.016, vol. 57, no. 11, 1 November 2008 (2008-11-01), pages 1591-1596, XP025532671, ISSN: 0026-0495 [retrieved on 2008-10-27]

## Description

### FIELD OF THE INVENTION

The invention relates to an ANGPTL4 polypeptide for use in the preservation of vascular endothelial cell barrier integrity and reduction in no-reflow phenomenon with myocardial infarction.

### BACKGROUND OF THE INVENTION

Heart disease represents the first and second causes of death in high- and low-income countries, respectively and treatments that minimize microvascular damage should be prioritized to protect the injured myocardium as recommended by the ACC/AHA guidelines. Rapid restoration of flow in the obstructed infarct artery, which can limit ischemia-induced tissue damage after the onset of ischemic symptoms, is a key determinant of short- and long-term outcomes for acute myocardial infarction (AMI) patients. Restoration of blood supply in cardiac ischemic tissue indeed restores oxygen and nutrients to starved myocardium and thus limits the extent of AMI but it also induces microvascular dysfunction, inflammation and oxidative damage.

Increased vascular permeability is an important contributor to edema and myocardial damage following ischemic events. Development of edema determines disruption of integrity which is detrimental to recovery and also permits extravasation of fibronectin and fibrinogen that form the provisional matrix network used by leukocytes for infiltrating. Vascular damage also contributes to the no-reflow phenomenom which is observed in 30% of patients with a reperfused anterior wall myocardial ischemia and is associated with a higher incidence of death. Leakiness of blood vessels in the heart therefore contributes to disease progression. Prevention of vascular leak and edema of endothelial and myocardial cells improves myocardial flow during reperfusion after acute myocardial infarction and its modulation in response to infarction represents a pertinent strategy to improve ongoing therapies that might limit infarct size, improve cardiac function and prolong survival. Yet, no pharmacological treatments currently target the no-reflow phenomenon.

Angiopoietins are major regulators of both vessel stabilisation and leakage through binding to the Tie2 receptor. Angiopoietin-like proteins (ANGPTL1-7) which belong to the angiopoietin family share some structural and functional properties with angiopoietins. ANGPTL4 is induced by hypoxia and produced by vascular cells in ischemic pathologies such as critical hind limb ischemia. ANGPTL4 is a 55-kDa secreted protein which is processed in a 20-kDa and a 35-kDa, comprising the coiled-coil domain and the fibrinogen-like domain, respectively. The coiled-coil domain mediates its oligomerization which is necessary for its activities. Studies characterizing the role of ANGPTL4 on angiogenesis provided complex results: survival factor or adhesion modulator of endothelial cells in vitro, and inducer as well as inhibitor of angiogenesis in different in vivo models that might depend on whether it is soluble or interacts with the extracellular matrix via heparan sulfate proteoglycans. ANGPTL4 also appears as a key modulator of lymphatic vessel development in the intestine and of blood vessel function through modulation of vascular permeability. Indeed, ANGPTL4 inhibits acute VEGF- or histamine-induced vascular hyperpermeability in the skin, as well as both intravasation and extravasation of metastatic tumor cells through inhibition of vascular permeability. In line with context-dependent activities of angiopoietins, ANGPTL4 induced increased extravasation of tumor cells in a TGF□-mediated lung metastasis mammary tumor model. ANGPTL4 also inhibits the lipoprotein (LPL) and hepatic (HL) lipases. In mouse, hepatic overexpression of ANGPTL4 induces hypertriglyceridemia whereas angptl4 genetic deletion or anti-ANGPTL4 antibody treatment causes lower plasma triglyceride levels. However, the role of ANGPTL4 in the regulation of lipid metabolism is not clear as angptl4 deleted mice displayed lower HDL levels compared to the control mice. In humans, ANGPTL4 possesses various variants, whom E40K which is associated with significant lower plasma triglycerides (TG) and higher levels of high-density lipoprotein cholesterol (HDL). Interestingly, although presenting an atheroprotective lipid profile, individuals carrying the ANGPTL4 inactive E40K variant are subjected to increased coronary heart disease risk. These recent data suggest that ANGPTL4 has a role in heart diseases through an as yet uncharacterized mechanism.

ITO Y ET AL: CANCER RESEARCH, AMERICAN ASSOCIATION FOR CANCER RESEARCH, BALTIMORE, MD., US,Vol. 63, no. 20, 15 October 2003 , pages 6651-6657; ITO Y ET AL: ARVO ANNUAL MEETING ABSTRACT SEARCH AND PROGRAM PLANNER,, 4 May 2003; GALAUP ARIANE ET AL; PROCEEDINGS OF THE NATIONAL ACADEMY OF SCIENCES OF THE UNITED STATES OF AMERICA, vol. 103, no. 49, December 2006, pages 18721-18726, disclose that ANGPTL4 polypeptide reduces vascular leakiness/permeability/angiogenesis. Therapeutic uses disclosed are the treatment of neoplastic diseases, diabetic retinopathy or metastasis. KIM I ET AL: BIOCHEMICAL JOURNAL, THE BIOCHEMICAL SOCIETY, LONDON, GB, vol. 346, no. 3, 15 March 2000 (2000-03-15), pages 603-610 discloses a protective effect of ANGPTL4 polypeptide on endothelial cells.

### SUMMARY OF THE INVENTION

The invention relates to an ANGPTL4 polypeptide for use in the prevention of no-reflow during the treatment of a coronary heart disease.

### DETAILED DESCRIPTION OF THE INVENTION

The inventors show that vascular permeability, hemorrhage, edema, inflammation and infarct severity are increased in angptl4-deficient mice. Post-ischemic decreased vegfr2 and ve-cadherin expression as well as increased Src kinase phosphorylation downstream of VEGFR2 were enhanced in the coronary microcirculation of angptl4-deficient mice. Both events led to altered VEGFR2/VE-Cadherin complex formation and to disrupted adherens junctions in endothelial cells of angptl4-deficient mice that correlated with an increased no-reflow. In contrast, recombinant human ANGPTL4 protected VEGF-driven VEGFR2/VE-Cadherin complex dissociation in endothelial cells and limited hemorrhages, reduced myocardial infarct size and the extent of no-reflow in rabbits. Accordingly, ANGPTL4-mediated vasculoprotection is crucial for secondary cardioprotection during AMI and might constitute a relevant target for therapeutic intervention.

### Therapeutic uses and methods according to the invention:

Disclosed herein is an ANGPTL4 polypeptide for use in the preservation of vascular endothelial cell barrier integrity.

As used herein, the term "vascular endothelial cell barrier" refers to the layer of cells that line the interior surface of blood vessels and act as a selective barrier between the vessel lumen and surrounding tissue, by controlling the transit of fluids, materials and cells such as myeloid cells and white blood cells into and out of the bloodstream. Excessive or prolonged increases in permeability of vascular endothelial cell barrier leads to tissue oedema/swelling. Accordingly the term "preservation of vascular endothelial cell barrier integrity" means the maintenance of the vascular endothelial cell barrier by avoiding or limiting permeability of said barrier.

More particularly, disclosed herein is an ANGPTL4 polypeptide for use in the preservation of vascular endothelial cell barrier integrity during treatment of ischemic conditions.

In the context of the invention, the term "treating" or "treatment", as used herein, means reversing and alleviating, inhibiting the progress of the ischemic condition to which such term applies condition. Typically, the treatment of ischemic conditions consists in the reperfusion of the ischemic organ (e.g. heart) by angioplasty (e.g.; coronary, renal or carotid angioplasty), thrombolysis or coronary surgery. The term "percutaneous coronary intervention (PCI)" means coronary angioplasty which is a therapeutic procedure to treat the stenotic (narrowed) coronary arteries of the heart found in coronary heart disease. The term "thrombolysis" means the administration of thrombolytic agents. Currently available thrombolyic agents include reteplase (r-PA or Retavase), alteplase (t-PA or Activase), urokinase (Abbokinase), prourokinase, anisoylated purified streptokinase activator complex (APSAC), and streptokinase.

The term "ischemic conditions" refers to any conditions that result from a restriction in blood supply in at least one organ or tissue. Theses conditions typically results from the obstruction of a blood vessel. For example ischemic conditions include but are not limited to renal ischemia, retinal ischemia, brain ischemia and myocardial ischemia. More particularly, the term includes but it is not limited to coronary artery bypass graft surgery, global cerebral ischemia due to cardiac arrest, focal cerebral infarction, cerebral hemorrhage, hemorrhage infarction, hypertensive hemorrhage, hemorrhage due to rupture of intracranial vascular abnormalities, subarachnoid hemorrhage due to rupture of intracranial arterial aneurysms, hypertensive encephalopathy, carotid stenosis or occlusion leading to cerebral ischemia, cardiogenic thromboembolism, spinal stroke and spinal cord injury, diseases of cerebral blood vessels: e.g., atherosclerosis, vasculitis, macular degeneration, myocardial infarction, cardiac ischemia and superaventicular tachyarrhytmia.

The invention provides an ANGPTL4 polypeptide for use in cardioprotection after acute myocardial infarction by preservation of vascular integrity that reduces infarct size, hemorrhage and no-reflow.

The invention relates to an ANGPTL4 polypeptide for use in the prevention of no-reflow in the treatment of coronary heart disease, especially myocardial infarction.

More particularly, the invention relates to an ANGPTL4 polypeptide for use in the prevention of no-reflow in the treatment of ST-segment elevation myocardial infarction.

The term "no-reflow" has been increasingly used in published medical reports to describe microvascular obstruction and reduced myocardial flow after opening an occluded artery. In its broadest meaning, the term "preventing no-reflow" or "prevention of no-reflow" refers to reducing or avoiding the no-reflow.

The term "patient" refers to any patient (preferably human) afflicted with an ischemic conditions.

In a particular option the patient is diagnosed with a coronary disorder, more particularly the patient has been diagnosed as presenting one of the following coronary disorders:
- chronic ischemic disorders without myocardial necrosis, such as stable or effort angina pectoris;
- acute ischemic disorders without myocardial necrosis, such as unstable angina pectoris;
- ischemic disorders with myocardial necrosis, such as ST segment elevation myocardial infarction or non-ST segment elevation myocardial infarction.

The term "ANGPTL4" has its general meaning in the art and refers to the Angiopoietin-like protein 4. ANGPTL4 is also named as HFARP (hepatic fibrinogen angiopoietin related protein), PGAR (PPARgamma angipoietin related protein) and FIAF (fasting induced adipose factor). The term includes naturally occurring ANGPTL4 and function conservative variants and modified forms thereof.

The ANGPTL4 can be from any source, but typically is a mammalian (e.g., human and non-human primate) ANGPTL4, and more particularly a human ANGPTL4. The sequence of ANGPTL4 protein and nucleic acids for encoding such proteins are well known to those of skill in the art. For example, Genbank Acc. Nos. NM_139314 and NM_001039667 and, Genbank Acc. Nos. AAH23647 provides the complete amino acid sequence of homo sapiens ANGPTL4. However, it should be understood that, as those of skill in the art are aware of the sequence of these molecules, any ANGPTL4 protein or gene sequence variant may be used as long as it has the properties of an ANGPTL4.

"Function conservative variants" are those in which a given amino acid residue in a protein or enzyme has been changed without altering the overall conformation and function of the polypeptide, including, but not limited to, replacement of an amino acid with one having similar properties (such as, for example, polarity, hydrogen bonding potential, acidic, basic, hydrophobic, aromatic, and the like). Amino acids other than those indicated as conserved may differ in a protein so that the percent protein or amino acid sequence similarity between any two proteins of similar function may vary and may be, for example, from 70 % to 99 % as determined according to an alignment scheme such as by the Cluster Method, wherein similarity is based on the MEGALIGN algorithm. A "function-conservative variant" also includes a polypeptide which has at least 60 % amino acid identity as determined by BLAST or FASTA algorithms, preferably at least 75 %, most preferably at least 85%, and even more preferably at least 90 %, and which has the same or substantially similar properties or functions as the native or parent protein to which it is compared.

According to the invention the term "ANGPTL4" polypeptide refers to any polypeptide that comprises the 20 k-Da N terminal fragment of ANGPTL4 (or function conservative thereof) and more specifically the N-terminal fragment coiled domain as described by Ge, H. et al. Oligomerization and regulated proteolytic processing of angiopoietin-like protein 4. J Biol Chem 279, 2038-45 (2004) ; Mandard, S. et al. The direct peroxisome proliferator-activated receptor target fasting-induced adipose factor (FIAF/PGAR/ANGPTL4) is present in blood plasma as a truncated protein that is increased by fenofibrate treatment. J Biol Chem 279, 34411-20 (2004) and ; Chomel, C. et al. Interaction of the coiled-coil domain with glycosaminoglycans protects angiopoietin-like 4 from proteolysis and regulates its antiangiogenic activity. Faseb J 23, 940-9 (2009). Accordingly, the term encompasses ANGPTL4 itself or fragments thereof comprising the 20 k-Da N terminal fragment.

In specific options, it is contemplated that ANGPTL4 polypeptides used in the therapeutic methods of the present invention may be modified in order to improve their therapeutic efficacy. Such modification of therapeutic compounds may be used to decrease toxicity, increase circulatory time, or modify biodistribution. For example, the toxicity of potentially important therapeutic compounds can be decreased significantly by combination with a variety of drug carrier vehicles that modify biodistribution.

A strategy for improving drug viability is the utilization of water-soluble polymers. Various water-soluble polymers have been shown to modify biodistribution, improve the mode of cellular uptake, change the permeability through physiological barriers; and modify the rate of clearance from the body. To achieve either a targeting or sustained-release effect, water-soluble polymers have been synthesized that contain drug moieties as terminal groups, as part of the backbone, or as pendent groups on the polymer chain.

Polyethylene glycol (PEG), has been widely used as a drug carrier, given its high degree of biocompatibility and ease of modification. Attachment to various drugs, proteins, and liposomes has been shown to improve residence time and decrease toxicity. PEG can be coupled to active agents through the hydroxyl groups at the ends of the chain and via other chemical methods; however, PEG itself is limited to at most two active agents per molecule. In a different approach, copolymers of PEG and amino acids were explored as novel biomaterials which would retain the biocompatibility properties of PEG, but which would have the added advantage of numerous attachment points per molecule (providing greater drug loading), and which could be synthetically designed to suit a variety of applications.

Those of skill in the art are aware of PEGylation techniques for the effective modification of drugs. For example, drug delivery polymers that consist of alternating polymers of PEG and tri-functional monomers such as lysine have been used by VectraMed (Plainsboro, N.J.). The PEG chains (typically 2000 daltons or less) are linked to the a- and e-amino groups of lysine through stable urethane linkages. Such copolymers retain the desirable properties of PEG, while providing reactive pendent groups (the carboxylic acid groups of lysine) at strictly controlled and predetermined intervals along the polymer chain. The reactive pendent groups can be used for derivatization, cross-linking, or conjugation with other molecules. These polymers are useful in producing stable, long-circulating pro-drugs by varying the molecular weight of the polymer, the molecular weight of the PEG segments, and the cleavable linkage between the drug and the polymer. The molecular weight of the PEG segments affects the spacing of the drug/linking group complex and the amount of drug per molecular weight of conjugate (smaller PEG segments provides greater drug loading). In general, increasing the overall molecular weight of the block co-polymer conjugate will increase the circulatory half-life of the conjugate. Nevertheless, the conjugate must either be readily degradable or have a molecular weight below the threshold-limiting glomular filtration (e.g., less than 45 kDa).

In addition, to the polymer backbone being important in maintaining circulatory half-life, and biodistribution, linkers may be used to maintain the therapeutic agent in a pro-drug form until released from the backbone polymer by a specific trigger, typically enzyme activity in the targeted tissue. For example, this type of tissue activated drug delivery is particularly useful where delivery to a specific site of biodistribution is required and the therapeutic agent is released at or near the site of pathology. Linking group libraries for use in activated drug delivery are known to those of skill in the art and may be based on enzyme kinetics, prevalence of active enzyme, and cleavage specificity of the selected disease-specific enzymes (see e.g., technologies of established by VectraMed, Plainsboro, N.J.). Such linkers may be used in modifying the ANGPTL4 polypeptides described herein for therapeutic delivery.

According to the invention, ANGPTL4 polypeptides may be produced by conventional automated peptide synthesis methods or by recombinant expression. General principles for designing and making proteins are well known to those of skill in the art.

ANGPTL4 polypeptides used in the invention may be synthesized in solution or on a solid support in accordance with conventional techniques. Various automatic synthesizers are commercially available and can be used in accordance with known protocols. ANGPTL4 polypeptides of the invention may also be synthesized by solid-phase technology employing an exemplary peptide synthesizer such as a Model 433A from Applied Biosystems Inc. The purity of any given protein; generated through automated peptide synthesis or through recombinant methods may be determined using reverse phase HPLC analysis. Chemical authenticity of each peptide may be established by any method well known to those of skill in the art.

As an alternative to automated peptide synthesis, recombinant DNA technology may be employed wherein a nucleotide sequence which encodes a protein of choice is inserted into an expression vector, transformed or transfected into an appropriate host cell and cultivated under conditions suitable for expression as described herein below. Recombinant methods are especially preferred for producing longer polypeptides.

A variety of expression vector/host systems may be utilized to contain and express the peptide or protein coding sequence. These include but are not limited to microorganisms such as bacteria transformed with recombinant bacteriophage, plasmid or cosmid DNA expression vectors; yeast transformed with yeast expression vectors; insect cell systems infected with virus expression vectors (e.g., baculovirus); plant cell systems transfected with virus expression vectors (e.g., cauliflower mosaic virus, CaMV; tobacco mosaic virus, TMV) or transformed with bacterial expression vectors (e.g., Ti or pBR322 plasmid); or animal cell systems. Those of skill in the art are aware of various techniques for optimizing mammalian expression of proteins. Mammalian cells that are useful in recombinant protein productions include but are not limited to VERO cells, HeLa cells, Chinese hamster ovary (CHO) cell lines, COS cells (such as COS-7), W138, BHK, HepG2, 3T3, RIN, MDCK, A549, PC12, K562 and 293 cells. Exemplary protocols for the recombinant expression of the peptide substrates or fusion polypeptides in bacteria, yeast and other invertebrates are known to those of skill in the art and a briefly described herein below. Mammalian host systems for the expression of recombinant proteins also are well known to those of skill in the art. Host cell strains may be chosen for a particular ability to process the expressed protein or produce certain post-translation modifications that will be useful in providing protein activity. Such modifications of the polypeptide include, but are not limited to, acetylation, carboxylation, glycosylation, phosphorylation, lipidation and acylation. Post-translational processing which cleaves a "prepro" form of the protein may also be important for correct insertion, folding and/or function. Different host cells such as CHO, HeLa, MDCK, 293, WI38, and the like have specific cellular machinery and characteristic mechanisms for such post-translational activities and may be chosen to ensure the correct modification and processing of the introduced, foreign protein.

In the recombinant production of the ANGPTL4 polypeptides of the invention, it would be necessary to employ vectors comprising polynucleotide molecules for encoding the ANGPTL4-derived proteins. Methods of preparing such vectors as well as producing host cells transformed with such vectors are well known to those skilled in the art. The polynucleotide molecules used in such an endeavor may be joined to a vector, which generally includes a selectable marker and an origin of replication, for propagation in a host. These elements of the expression constructs are well known to those of skill in the art. Generally, the expression vectors include DNA encoding the given protein being operably linked to suitable transcriptional or translational regulatory sequences, such as those derived from a mammalian, microbial, viral, or insect genes. Examples of regulatory sequences include transcriptional promoters, operators, or enhancers, mRNA ribosomal binding sites, and appropriate sequences which control transcription and translation.

The terms "expression vector," "expression construct" or "expression cassette" are used interchangeably throughout this specification and are meant to include any type of genetic construct containing a nucleic acid coding for a gene product in which part or all of the nucleic acid encoding sequence is capable of being transcribed.

The choice of a suitable expression vector for expression of the peptides or polypeptides of the invention will of course depend upon the specific host cell to be used, and is within the skill of the ordinary artisan. Methods for the construction of mammalian expression vectors are disclosed, for example, in EP-A-0367566; and WO 91/18982.

In general, the vectors useful in the invention include, but are not limited to, plasmids, phagemids, viruses, other vehicles derived from viral or bacterial sources that have been manipulated by the insertion or incorporation of the antisense oligonucleotide, siRNA, shRNA or ribozyme nucleic acid sequences. Viral vectors are a preferred type of vector and include, but are not limited to nucleic acid sequences from the following viruses: retrovirus, such as moloney murine leukemia virus, harvey murine sarcoma virus, murine mammary tumor virus, and rous sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known to the art.

Preferred viral vectors are based on non-cytopathic eukaryotic viruses in which non-essential genes have been replaced with the gene of interest. Non-cytopathic viruses include retroviruses (e.g., lentivirus), the life cycle of which involves reverse transcription of genomic viral RNA into DNA with subsequent proviral integration into host cellular DNA. Retroviruses have been approved for human gene therapy trials. Most useful are those retroviruses that are replication-deficient (i.e., capable of directing synthesis of the desired proteins, but incapable of manufacturing an infectious particle). Such genetically altered retroviral expression vectors have general utility for the high-efficiency transduction of genes in vivo. Standard protocols for producing replication-deficient retroviruses (including the steps of incorporation of exogenous genetic material into a plasmid, transfection of a packaging cell lined with plasmid, production of recombinant retroviruses by the packaging cell line, collection of viral particles from tissue culture media, and infection of the target cells with viral particles) are well known in the art.

Preferred viruses for certain applications are the adenoviruses and adeno-associated (AAV) viruses, which are double-stranded DNA viruses that have already been approved for human use in gene therapy. Actually 12 different AAV serotypes (AAV1 to 12) are known, each with different tissue tropisms. Recombinant AAV are derived from the dependent parvovirus AAV2. The adeno-associated virus type 1 to 12 can be engineered to be replication deficient and is capable of infecting a wide range of cell types and species. It further has advantages such as, heat and lipid solvent stability; high transduction frequencies in cells of diverse lineages, including hemopoietic cells; and lack of superinfection inhibition thus allowing multiple series of transductions. Reportedly, the adeno-associated virus can integrate into human cellular DNA in a site-specific manner, thereby minimizing the possibility of insertional mutagenesis and variability of inserted gene expression characteristic of retroviral infection. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion.

Other vectors include plasmid vectors. Plasmid vectors have been extensively described in the art and are well known to those of skill in the art. In the last few years, plasmid vectors have been used as DNA vaccines for delivering antigen-encoding genes to cells in vivo. They are particularly advantageous for this because they do not have the same safety concerns as with many of the viral vectors. These plasmids, however, having a promoter compatible with the host cell, can express a peptide from a gene operatively encoded within the plasmid. Some commonly used plasmids include pBR322, pUC18, pUC19, pRC/CMV, SV40, and pBlueScript. Other plasmids are well known to those of ordinary skill in the art. Additionally, plasmids may be custom designed using restriction enzymes and ligation reactions to remove and add specific fragments of DNA. Plasmids may be delivered by a variety of parenteral, mucosal and topical routes. For example, the DNA plasmid can be injected by intramuscular, intradermal, subcutaneous, or other routes. It may also be administered by intranasal sprays or drops, rectal suppository and orally. It may also be administered into the epidermis or a mucosal surface using a gene-gun. The plasmids may be given in an aqueous solution, dried onto gold particles or in association with another DNA delivery system including but not limited to liposomes, dendrimers, cochleate and microencapsulation.

Expression requires that appropriate signals be provided in the vectors, such as enhancers/promoters from both viral and mammalian sources that may be used to drive expression of the nucleic acids of interest in host cells. Usually, the nucleic acid being expressed is under transcriptional control of a promoter. A "promoter" refers to a DNA sequence recognized by the synthetic machinery of the cell, or introduced synthetic machinery, required to initiate the specific transcription of a gene. Nucleotide sequences are operably linked when the regulatory sequence functionally relates to the DNA encoding the protein of interest (i.e., ANGPTL4, a variant and the like). Thus, a promoter nucleotide sequence is operably linked to a given DNA sequence if the promoter nucleotide sequence directs the transcription of the sequence.

Similarly, the phrase "under transcriptional control" means that the promoter is in the correct location and orientation in relation to the nucleic acid to control RNA polymerase initiation and expression of the gene. Any promoter that will drive the expression of the nucleic acid may be used. The particular promoter employed to control the expression of a nucleic acid sequence of interest is not believed to be important, so long as it is capable of directing the expression of the nucleic acid in the targeted cell. Thus, where a human cell is targeted, it is preferable to position the nucleic acid coding region adjacent to and under the control of a promoter that is capable of being expressed in a human cell. Generally speaking, such a promoter might include either a human or viral promoter. Common promoters include, e.g., the human cytomegalovirus (CMV) immediate early gene promoter, the SV40 early promoter, the Rous sarcoma virus long terminal repeat, [beta]-actin, rat insulin promoter, the phosphoglycerol kinase promoter and glyceraldehyde-3-phosphate dehydrogenase promoter, all of which are promoters well known and readily available to those of skill in the art, can be used to obtain high-level expression of the coding sequence of interest. The use of other viral or mammalian cellular or bacterial phage promoters which are well-known in the art to achieve expression of a coding sequence of interest is contemplated as well, provided that the levels of expression are sufficient to produce a recoverable yield of protein of interest. By employing a promoter with well known properties, the level and pattern of expression of the protein of interest following transfection or transformation can be optimized. Inducible promoters also may be used.

Another regulatory element that is used in protein expression is an enhancer. These are genetic elements that increase transcription from a promoter located at a distant position on the same molecule of DNA. Where an expression construct employs a cDNA insert, one will typically desire to include a polyadenylation signal sequence to effect proper polyadenylation of the gene transcript. Any polyadenylation signal sequence recognized by cells of the selected transgenic animal species is suitable for the practice of the invention, such as human or bovine growth hormone and SV40 polyadenylation signals.

Another aspect of the disclosure relates to a nucleic acid molecule encoding for an ANGPTL4 polypeptide for use in the preservation of vascular endothelial cell barrier integrity as above described.

Typically, said nucleic acid is a DNA or RNA molecule, which may be included in any suitable vector, such as a plasmid, cosmid, episome, artificial chromosome, phage or a viral vector as above described.

So, a further object of the disclosure relates to a vector comprising a nucleic acid encoding for an ANGPTL4 polypeptide for use in the preservation of vascular endothelial cell barrier integrity as above described.

A further object of the disclosure relates to a host cell comprising a nucleic acid encoding for an ANGPTL4 polypeptide (or a vector comprising a nucleic acid thereof) for the preservation of vascular endothelial cell barrier integrity as above described.

The same nucleic acids and vectors also may be useful for use in the prevention of no-reflow in the treatment of coronary heart disease, especially myocardial infarction (not claimed.)

The present invention provides a method treating an ischemic condition in a patient in need thereof comprising the step of consisting of:
- i) restoring blood supply the ischemic organ or tissue
- ii) preserving the vascular endothelial cell barrier integrity of said ischemic organ or tissue by administrating said patient with a therapeutically effective amount of ANGPTL4 polypeptides (or nucleic acids encoding for ANGPTL4 polypeptides or a vectors comprising a nucleic acids encoding for ANGPTL4 polypeptides not claimed ..) according to the invention.

More particularly the present invention provides a method for treating acute myocardial infarction in a patient in need thereof comprising the steps consisting of:
- i) restoring blood supply in the cardiac ischemic tissue
- ii) preserving the vascular endothelial cell barrier integrity of said cardiac ischemic tissue by administrating said patient with therapeutically effective amount of ANGPTL4 polypeptides (or nucleic acids encoding for ANGPTL4 polypeptides or a vectors comprising a nucleic acids encoding for ANGPTL4 polypeptides not claimed) according to the invention.

In particular option, steps i) and ii) as above described may performed sequentially or concomitantly.

By a "therapeutically effective amount" is meant a sufficient amount of ANGPTL4 polypeptides (or nucleic acid encoding for a ANGPTL4 polypeptide or a vector comprising a nucleic acid encoding for a ANGPTL4 polypeptide not claimed) to preserve the vascular endothelial cell barrier integrity at a reasonable benefit/risk ratio applicable to any medical treatment. It will be understood that the total usage of the compounds and compositions of the present invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination or coincidental with the specific polypeptide employed; and like factors well known in the medical arts. For example, it is well known within the skill of the art to start doses of the compound at levels lower than those required to achieve the desired therapeutic effect and to gradually increase the dosage until the desired effect is achieved.

### Pharmaceutical compositions according to the invention:

A further object of the invention relates to pharmaceutical compositions comprising a ANGPTL4 polypeptide (or a vector comprising a nucleic acid encoding for a ANGPTL4 polypeptide not claimed...) for use in the preservation of vascular endothelial cell barrier integrity as above described. The pharmaceutical compositions according to the invention are useful for use in the prevention of no-reflow in the treatment of coronary heart disease, especially myocardial infarction.

Typically, the ANGPTL4 polypeptide (or nucleic acid encoding for a ANGPTL4 polypeptide or a vector comprising a nucleic acid encoding for a ANGPTL4 polypeptide; not claimed) may be combined with pharmaceutically acceptable excipients, and optionally sustained-release matrices, such as biodegradable polymers, to form therapeutic compositions.

"Pharmaceutically" or "pharmaceutically acceptable" refer to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

In the pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The ANGPTL4 polypeptide (or nucleic acid encoding for a ANGPTL4 polypeptide or a vector comprising a nucleic acid encoding for a ANGPTL4 polypeptide; not claimed) can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active polypeptides in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. Some variation in dosage will necessarily occur depending on the condition of the patient being treated. The person responsible for administration will, in any event, determine the appropriate dose for the individual patient.

In addition to the compounds of the invention formulated for parenteral administration, such as intravenous or intramuscular injection, other pharmaceutically acceptable forms include, e.g. tablets or other solids for oral administration; liposomal formulations; time release capsules; and any other form currently used.

### Medical devices according to the invention:

The present invention also provides the use of an ANGPTL4 polypeptide (or nucleic acid encoding for a ANGPTL4 polypeptide or a vector comprising a nucleic acid encoding for a ANGPTL4 polypeptide; unclaimed) for the preparation of biomaterials or medical delivery devices selected among endovascular prostheses, such as stents, bypass grafts, internal patches around the vascular tube, external patches around the vascular tube, vascular cuff, and angioplasty catheter.

In this respect, the invention relates more particularly to biomaterials or medical delivery devices as mentioned above, coated with such ANGPTL4 polypeptide (or nucleic acid encoding for a ANGPTL4 polypeptide or a vector comprising a nucleic acid encoding for a ANGPTL4 polypeptide; unclaimed) expression and/or activity as defined above, said biomaterials or medical devices being selected among endovascular prostheses, such as stents, bypass grafts, internal patches around the vascular tube, external patches around the vascular tube, vascular cuff, and angioplasty catheter. Such a local biomaterial or medical delivery device can be used to reduce stenosis as an adjunct to revascularizing, bypass or grafting procedures performed in any vascular location including coronary arteries, carotid arteries, renal arteries, peripheral arteries, cerebral arteries or any other arterial or venous location, to reduce anastomic stenosis such as in the case of arterial-venous dialysis access with or without polytetrafluoro- ethylene grafting and with or without stenting, or in conjunction with any other heart or transplantation procedures, or congenital vascular interventions.

For illustration purpose, such endovascular prostheses and methods for coating ANGPTL4 polypetides thereto are more particularly described in WO2005094916, or are those currently used in the art. The compounds used for the coating of the prostheses should preferentially permit a controlled release of said inhibitor. Said compounds could be polymers (such as sutures, polycarbonate, Hydron, and Elvax), biopolymers/biomatrices (such as alginate,fucans, collagen-based matrices, heparan sulfate) or synthetic compounds such as synthetic heparan sulfate-like molecules or combinations thereof. Other examples of polymeric materials may include biocompatible degradable materials, e. g. lactone-based polyesters orcopolyesters, e. g. polylactide ; polylactide-glycolide ;polycaprolactone- glycolide ; polyorthoesters ; polyanhydrides ; polyaminoacids ; polysaccharides ;polyphospha- zenes; poly (ether-ester) copolymers, e. g. PEO-PLLA, or mixtures thereof; and biocompatible non-degrading materials, e. g. polydimethylsiloxane ; poly (ethylene-vinylacetate) ; acrylate based polymers or coplymers, e. g. polybutylmethacrylate, poly (hydroxyethyl methylmethacrylate) ; polyvinyl pyrrolidinone ;fluorinated polymers such as polytetrafluoethylene ; cellulose esters. When a polymeric matrix is used, it may comprise 2 layers, e. g. a base layer in which said inhibitor is incorporated, such as ethylene-co-vinylacetate and polybutylmethacrylate, and a top coat, such as polybutylmethacrylate, which acts as a diffusion-control of said inhibitor. Alternatively, said inhibitor may be comprised in the base layer and the adjunct may be incorporated in the outlayer, or vice versa.

Such biomaterial or medical delivery device may be biodegradable or may be made of metal or alloy, e. g. Ni and Ti, or another stable substance when intented for permanent use. The inhibitor of the invention may also be entrapped into the metal of the stent or graft body which has been modified to contain micropores or channels. Also internal patches around the vascular tube, external patches around the vascular tube, or vascular cuff made of polymer or other biocompatible materials as disclosed above that contain the inhibitor of the invention may also be used for local delivery.

Said biomaterial or medical delivery device allow the inhibitor releasing from said biomaterial or medical delivery device over time and entering the surrounding tissue. Said releasing may occur during 1 month to 1 year. The local delivery according to the present invention allows for high concentration of the inhibitor of the invention at the disease site with low concentration of circulating compound. The amount of said inhibitor used for such local delivery applications will vary depending on the compounds used, the condition to be treated and the desired effect. For purposes of the invention, a therapeutically effective amount will be administered.

The local administration of said biomaterial or medical delivery device preferably takes place at or near the vascular lesions sites. The administration may be by one or more of the following routes: via catheter or other intravascular delivery system, intranasally, intrabronchially, interperitoneally or eosophagal. Stents are commonly used as a tubular structure left inside the lumen of a duct to relieve an obstruction. They may be inserted into the duct lumen in a non-expanded form and are then expanded autonomously (self-expanding stents) or with the aid of a second device in situ, e. g. a catheter-mounted angioplasty balloon which is inflated within the stenosed vessel or body passageway in order to shear and disrupt the obstructions associated with the wall components of the vessel and to obtain an enlarged lumen.

### Diagnostic methods of the invention:

A further aspect of the disclosure relates to a method of testing a patient thought to have or be predisposed to microvascular dysfunction, which comprises the step of analyzing a biological sample from said patient for:
(i) detecting the presence of a mutation in the gene encoding for ANGPTL4 and/or its associated promoter, and/or
(ii) analyzing the expression of the gene encoding for ANGPTL4.

As used herein, the term "biological sample" refers to any sample from a patient such as blood or serum.

Typical techniques for detecting a mutation in the gene encoding for ANGPTL4 may include restriction fragment length polymorphism, hybridisation techniques, DNA sequencing, exonuclease resistance, microsequencing, solid phase extension using ddNTPs, extension in solution using ddNTPs, oligonucleotide assays, methods for detecting single nucleotide polymorphism such as dynamic allele-specific hybridisation, ligation chain reaction, mini-sequencing, DNA "chips", allele-specific oligonucleotide hybridisation with single or dual-labelled probes merged with PCR or with molecular beacons, and others.

Analyzing the expression of the gene encoding for ANGPTL4 may be assessed by any of a wide variety of well-known methods for detecting expression of a transcribed nucleic acid or translated protein.

In a preferred option the expression of the gene encoding for ANGPTL4 is assessed by analyzing the expression of mRNA transcript or mRNA precursors, such as nascent RNA, of said gene. Said analysis can be assessed by preparing mRNA/cDNA from cells in a biological sample from a patient, and hybridizing the mRNA/cDNA with a reference polynucleotide. The prepared mRNA/cDNA can be used in hybridization or amplification assays that include, but are not limited to, Southern or Northern analyses, polymerase chain reaction analyses, such as quantitative PCR (TaqMan), and probes arrays such as GeneChip(TM) DNA Arrays (AFF YMETRIX).

Advantageously, the analysis of the expression level of mRNA transcribed from the gene encoding for ANGPTL4 involves the process of nucleic acid amplification, e. g., by RT-PCR (the experimental embodiment set forth in U. S. Patent No. 4,683, 202), ligase chain reaction (BARANY, Proc. Natl. Acad. Sci. USA, vol.88, p: 189-193, 1991), self sustained sequence replication (GUATELLI et al., Proc. Natl. Acad. Sci. USA, vol.57, p: 1874-1878, 1990), transcriptional amplification system (KWOH et al., 1989, Proc. Natl. Acad. Sci. USA, vol.86, p: 1173-1177, 1989), Q-Beta Replicase (LIZARDI et al., Biol. Technology, vol.6, p: 1197, 1988), rolling circle replication (U. S. Patent No. 5,854, 033) or any other nucleic acid amplification method, followed by the detection of the amplified molecules using techniques well known to those of skill in the art. These detection schemes are especially useful for the detection of nucleic acid molecules if such molecules are present in very low numbers. As used herein, amplification primers are defined as being a pair of nucleic acid molecules that can anneal to 5 'or 3 'regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

In another preferred option, the expression of the gene encoding for ANGPTL4 is assessed by analyzing the expression of the protein translated from said gene. Said analysis can be assessed using an antibody (e.g., a radio-labeled, chromophore- labeled, fluorophore-labeled, or enzyme-labeled antibody), an antibody derivative (e.g., an antibody conjugate with a substrate or with the protein or ligand of a protein of a protein/ligand pair (e.g., biotin-streptavidin)), or an antibody fragment (e.g., a single-chain antibody, an isolated antibody hypervariable domain, etc.) which binds specifically to the protein translated from the gene encoding for ANGPTL4.

Said analysis can be assessed by a variety of techniques well known from one of skill in the art including, but not limited to, enzyme immunoassay (EIA), radioimmunoassay (RIA), Western blot analysis and enzyme linked immunoabsorbant assay (RIA).

The method may comprise comparing the level of expression of the gene encoding for ANGPTL4 in a biological sample from a patient with the normal expression level of said gene in a control. A significantly weaker level of expression of said gene in the biological sample of a patient as compared to the normal expression level is an indication that the patient has or is predisposed to developing micovascular dysfunction. The "normal" level of expression of the gene encoding for ANGPTL4 is the level of expression of said gene in a biological sample of a patient not afflicted by microvascular dysfunction. Preferably, said normal level of expression is assessed in a control sample (e.g., sample from a healthy patient, which is not afflicted by an increased retinal vascular permeability) and preferably, the average expression level of said gene in several control samples.

The invention will be further illustrated by the following figures and examples. However, these examples and figures should not be interpreted in any way as limiting the scope of the present invention.

### FIGURES

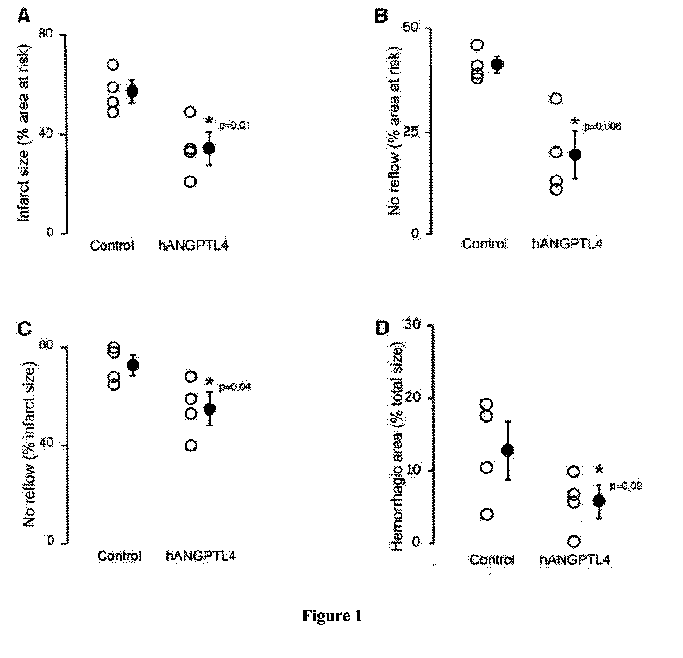

**Figure 1** **:** Infarct sizes (expressed as a percentage of the area at risk) in control rabbits and in 100 ng/ml human recombinant ANGPTL4 injected-rabbits. Open circles represent individual infarct size and closed circles represent mean± SEM. *p<0.05 (A). Extent of the no-reflow zone expressed as a percentage of infarct size (B) or expressed as a percentage of the area at risk (C) in control rabbits and in 100 ng/ml human recombinant ANGPTL4 injected-rabbits. Open circles represent individual no-reflow zone and closed circles represent mean± SEM. *p<0.05. Microscopic images of rabbit infarcted myocardium (D, scale bar=250µm). HE staining revealed diffused ischemic areas characterized by hemorrhages shown by arrow (A) which were more prominent in control rabbits compared to 100 ng/ml human recombinant ANGPTL4 injected-rabbits.

### EXAMPLE 1 : CARDIOPROTECTION THROUGH PRESERVATION OF VASCULAR ENDOTHELIAL CELL BARRIER INTEGRITY BY ANGIOPOIETIN-LIKE 4

### Material & methods:

The experiments were performed in accordance with the official regulations edited by the French Ministry of Agriculture. This study conforms to the standards of INSERM (the French National Institute of Health) regarding the care and use of laboratory animals, was performed in accordance with European Union Council Directives (86/609/EEC).

**Animals and genotyping:** Genotype was determined by PCR of tail genomic DNA using the following conditions : denaturation at 94°C for 30 seconds, annealing at 56°C for 45 seconds, and extension at 72°C for 1 minute and 15 seconds, 30 cycles.. Wild-type (*angptl4*^{+/+})*, angptl4*^{LacZ/+} and *angptl4^{LacZlLacZ}* knock-out C57BL/6 mice, 8 to 12 weeks of age, were subjected to myocardial infarction protocols or used as control in basal conditions.

**Myocardial ischemia-reperfusion experiments:** Male *angptl4*^{LacZ/+} and *angptl4*^{*LacZ*/*LacZ*} mice were anesthetized by an intraperitoneal injection of sodium pentobarbital. Myocardial infarction with transient occlusion of the left coronary artery was performed for 45mn and then tissues were reperfused for 1h to 3 weeks. For *angptl4* expression study WT mice underwent 1h, 3h, 24h, 48h, 72h, 1 week, 2 weeks, 3 weeks of reperfusion (2 animals per group). To assess infarct size and for immunohistochemistry (IHC) or ultrastructural studies, male *angptl4*^{LacZ/+} and *angptl4*^{*LacZ*/*LacZ*} mice were reperfused during either 4h or 48h after ischemia (4 or 5 animals per group). For permeability analyses, only 4h of reperfusion were performed with *angptl4*^{LacZ/+} and *angptl4*^{*LacZ*/*LacZ*} mice (3 to 5 animals per group). The area at risk was identified by Evans blue staining, and the infarct area was identified by 2,3,5-triphenyltetrazolium chloride (TTC) staining. The area at risk was identified as the nonblue region and expressed as a percentage of the left ventricle weight. The infarcted area was identified as the TTC-negative zone and expressed as a percentage of the area at risk. Ultrastructural analyses were performed on a Hitachi H-9500 electron microscope.

**Rabbits experiments** :New Zealand rabbits (2.5-3.0 kg) were anesthetized using zolazepam, tiletamine and pentobarbital (all 20-30 mg/kg i.v.). The animals were intubated, mechanically ventilated and a left thoracotomy was performed. A suture was passed beneath a major branch of the left coronary artery. The ends of the ligature were passed through a short propylene tubing to form a snare. Rabbits then randomly received either vehicle or human recombinant angptl4 (10µg/kg i.v.; 100ng/ml). Five minutes after, coronary artery occlusion (CAO) was induced during 30-min by pulling the snare through the tubing. Reperfusion was subsequently induced by releasing the snare. The chest was then closed in layers. Four hours after the onset of reperfusion, the chest was reopened and thioflavine S (4%; 1.5 ml/kg) was rapidly infused through the left atrium. Rabbits were then sacrificed using pentobarbital followed by potassium chloride. After excision, the hearts were perfused retrogradely with Alcian blue (0.5%) and cut into slices. Slices were photographed using UV light to identify the region of no-reflow. The areas of no-reflow, infarct and risk zone were determined as in mice.

***In situ* hybridization (ISH) and immunohistochemistry (IHC) analysis:** Full length human or mouse *angptl4* ORF was amplified by PCR, subcloned in pGem.T (Promega) and used to generate a ³⁵S-RNA antisense mouse *angptl4* probe. A sense probe was used as a negative control.and used as a template. ISH using sense probe, antisense human or mouse *angptl4* probes and IHC immunolabellings anti-CD45, -Mac3, and -CD31 experiments were performed as previously described (Brechot, N. et al. Modulation of macrophage activation state protects tissue from necrosis during critical limb ischemia in thrombospondin-1-deficient mice. PLoS ONE 3, e3950 (2008)).. ISH detecting human *angptl4* combined to IHC using anti-CD34 antibody (R&D system) were performed on human infarcted hearts sections.

**Modified Miles assay:** Male *angptl4*^{*-*/*-*} and *angptl4*^{+/-} mice were anesthetized with pentobarbital. For basal conditions, mice were injected into the tail vein with 1% Evans blue (200µl) and sacrificed 4h later. For the ischemia-reperfusion conditions, mice were subjected to coronary occlusion for 45mn and intravenously injected with 1% Evans blue (200µl) prior the 4h of reperfusion. At sacrifice, mice were perfused through the aorta with citrate buffer pH4 using a 18-gauge cannula. Blood, dye and buffer exited through an opening in the right atrium. Evans Blue was eluted for 18h at 70°C in 1ml formamide. After centrifugation, absorbance at 620 nm was measured using a spectrophotometer. Extravasated Evans blue (ng) was determined from a standard curve and normalized to tissue weight (g). Data are presented as mean ± s.e.m. of 3 or 5 mice per genotype.

**Immunofluoresence study and confocal analyzis on cryosections:** Mice subjected to ischemia and 4h of reperfusion were anesthetized with ketamine and xylazine injected intraperitonally. FITC-beads (20µl) were injected into the femoral vein as previously described (Baffert, F., Le, T., Thurston, G. & McDonald, D.M. Angiopoietin-1 decreases plasma leakage by reducing number and size of endothelial gaps in venules. Am J Physiol Heart Circ Physiol 290, H107-18 (2006)).. The chest was opened rapidly, and the vasculature was perfused for 2 minutes at a pressure of 120 mmHg with 1% paraformaldehyde as described for the modified Miles assay. The heart was then placed into 1% paraformaldehyde for 1h at room temperature, rinsed several times with PBS, infiltrated overnight with 30% sucrose, and frozen for cryostat sectioning. Sections 80 µm in thickness were incubated in 5% normal goat serum in PBS containing 0.3% Triton X-100, 0.2% bovine serum albumin, for 1 hour at RT to block nonspecific antibody binding and were incubated O/N with the primary antibodies. Endothelial cells, pericytes and adherens junctions were identified with rat anti-CD31 (BD Pharmingen), rabbit anti-NG2 (Chemicon) and rat anti-VE-Cadherin (personal gift from E. Dejana, IFOM) antibodies, respectively. After several rinses with PBS, specimens were incubated for 3h at room temperature with fluorescent (Alexa 555 and Alexa 647) secondary antibodies (anti-rat or anti-rabbit; Molecular Probes) diluted 1:500. Finally specimens were mounted in Vectashield (Vector Laboratories). Confocal images were acquired by using a Leica TCS SP5 microscope. Confocal sections were imaged on a Leica SP5 microscope (Leica Microsystems GmbH) using a 63x (NA = 1.4) oil objective. An increment of 0.117 µm between each section was used. 3D reconstruction of the different structures were obtained using the LABELVOXEL and the SURFACEGEN modules in Amira 5.2.1 software (Visage Imaging GmbH).

**Isolation of cardiomyocytes and viability assay** :Under anesthesia, the heart was removed from the chest and was cannulated. The heart was perfused for 4 min with tyrode buffer ([mM] NaCl 113; KCl 4.7 ; KH₂PO₄ 0.6 ; Na₂HPO₄ 0.6 ; HEPES 10 ; MgSO₄ 1.2 ; NaHCO₃ 12 ; KHCO₃ 10 ; taurine 30 ; phenol red 0.032 ; glucose 5.5 ; with pH ajusted to 7.46 with NaOH 1N) at constant pressure and 37°C. Perfusion was switched to an enzyme solution ([mM] NaCl 113 ; KCl 4.7 ; KH₂PO₄ 0.6 ; Na₂HPO₄ 0.6 ; HEPES 10 ; MgSO₄ 1.2 ; NaHCO₃ 12 ; KHCO₃ 10 ; taurine 30 ; phenol red 0.032 ; glucose 5.5 ; CaCl₂ 0.0125 ; with pH ajusted to 7.46 with NaOH 1N) containing 0.1mg/ml liberase blendzyme IV, (Roche diagnostics) and 0.14mg/ml trypsin (Sigma). When hearts became swollen and turn slightly pale, the atria and aorta were removed; the left ventricle were cut into small pieces and gently triturated. Cell suspension was transferred into a stopping buffer ([mM] NaCl 113 ; KCl 4.7 ; KH₂PO₄ 0.6 ; Na₂HPO₄ 0.6 HEPES 10 ; MgSO₄ 1.2 ; NaHCO₃ 12 ; KHCO₃ 10 ; taurine 30 ; phenol red 0.032 ; glucose 5.5 ; CaCl₂ 0.0125 ; calf serum 5% ; with pH adjusted to 7.46 with NaOH 1N). Extracellular calcium was added incrementally up to 1.0 mM. All cells studied were rod-shaped, had clear cross-striations and lacked any visible vesicles on their surfaces under observations with an optical microscope. Cardiac myocytes were incubated in an anaerobic chamber containing a humidified atmosphere of 5% CO₂ and 95% N₂ for 3h. Experimental medium was changed to serum-free, glucose-free. Normoxic experimental medium was equilibrated in water-jacketed incubators in a humidified atmosphere of 5% CO₂ and air. Cardiac myocyte survival was measured by staining cells in tissue culture dishes with trypan blue solution (Sigma, Saint-Quentin Fallavier, France) diluted to a final concentration of 0.4%. Myocytes were visualized using brightfield microscopy at 100x magnification. The number of viable (unstained) and non-viable (blue stained) cardiac myocytes in three random microscopic fields was recorded, and at least 100 cells were counted in each field. Percent survival was defined as the number of unstained myocytes counted in each dish divided by the number of total (unstained and stained) myocytes in each condition.

**Ultrasound analyzis of cardiac parameters:** Mice were subjected to ultrasound measurements using an echocardiograph (Vivid 7, GE Medical Systems Ultrasound) equipped with a 12-MHz linear transducer.

**Statistical analyses:** Analyses were performed using StatView Software (version 5.0; Abacus Concepts, Inc., Berkeley, CA).

### Results :

**ANGPTL4 expression after myocardial infarction in humans :** As ANGPTL4 is induced by hypoxia and expressed in different pathological conditions in humans, such as critical hindlimb ischemia and in numerous types of tumours, we here sought to investigate whether ANGPTL4 is expressed in the human heart after myocardial ischemia. Tissue sections from patients who died from an acute myocardial infarction, obtained from the Pathology Department of Georges Pompidou European Hospital, Paris, France, allowed to visualize the infarcted area. Using ISH, *angptl4* mRNA expression was analyzed in following sections. A patchy staining pattern surrounding the infarcted areas was observed. Analysis at higher magnification showed *angptl4* mRNA expression was induced in cardiomyocytes, in mononuclear inflammatory cells, likely macrophages and in endothelial cells positive for CD34.

**Infarct size and tissue damages are increased in *angptl4* ^{*LacZ*/*LacZ*} mice:** *angptl4* mRNA expression was then analyzed in a mouse model of myocardial ischemia reperfusion in C57/B16 mice. It could be detected *in situ* in the infarcted area by ISH as soon as 3h following ischemia and up to 2 weeks after reperfusion. To determine the functional role that ANGPTL4 might play during myocardial infarction, *angptl4* knockout mice in which the *angptl4* locus was replaced by a *lacZ* reporter gene were generated and intercrossed in C57/B16 mice for more than 8 generations. Some lethality was observed during development but surviving *angptl4^{LacZlLacZ}* neonates, which were obtained at ∼25% of the expected frequency, were viable and fertile and did not exhibit functional cardiac defects.

Myocardial ischemia-reperfusion was performed in *angptl4*^{LacZ/+} and in *angptl4*^{*LacZ*/*LacZ*} mice. Infarct size expressed as a percentage of the risk zone (IS, % area at risk) was increased in *angptl4*^{LacZ/*LacZ*} mice (47± 3%) compared to control mice (36± 3%).

We then quantified hemorrhage, edema, inflammation on HE slides from infarcted heart sections in both genotypes. In accordance with increased infarct size, histological analyses of HE-stained sections revealed that tissue necrosis was largely increased in *angptl4*^{*LacZ*/*LacZ*} compared to control mice (histological score of 2.5 in *angptl4*^{*LacZ*/}*^{LacZ} versus* 1.2 in *angptl4*^{*LacZ*/+} mice). A more severe tissue injury in *angptl4*^{*LacZ*/*LacZ*} compared to *angptl4*^{*LacZ*/+} mice was also quantified in term of edema (2.3 *versus* 0.8), hemorrhages (2.3 *versus* 1.1) and inflammation (2.8 *versus* 1.1).

**Post-ischemic inflammation but not capillary density is modulated in *angptl4*^{*LacZ*/*LacZ*} mice:** As post-ischemic inflammation response may also influence tissue damage, we then analyzed macrophage density using Mac3 immunostainings both in infarcted and non-infarcted areas delineated on adjacent HE-stained sections, in both genotypes. Macrophage density was significantly higher in infarcted areas in *angptl4*^{*LacZ*/}*^{LacZ} versus angptl4*^{*LacZ*/+} mice (mean macrophage density reached 1822± 117 *versus* 438± 71/mm², p=0.017) whereas no statistical difference was observed between both groups in control non-infarcted areas (157± 120 *versus* 111± 48 /mm², p=0.33).

Adult cardiac vascular network was also analyzed using CD31 and no difference was quantified between both genotypes neither in basal conditions nor in the infarcted areas (4510± 124 *versus* 4380± 117 mean capillary density /mm², respectively, p=0.22). In the most altered infarcted areas (central infarcted areas), a decreased microcapillary density was similarly observed in both genotypes (mean capillary density reached 1115± 56 in *angptl4*^{*LacZ*/}*^{LacZ} versus* 1030± 48 /mm² in *angptl4*^{*LacZ*/+}*,* p=0.16).

**Cardiomyocyte viability from *angptl4*^{*LacZ*/*LacZ*} mice is not affected by hypoxia:** ***In** vitro* and *in vivo* experiments were then performed in order to decipher which mechanisms might be responsible for increased infarct size and tissue damages in *angptl4^{LacZlLacZ}* mice. As *angptl4* mRNA was induced by hypoxia mediated by hypoxia-inducible factor 1 in cardiomyocytes, we first hypothezised a direct autocrine effect of ANGPTL4. Cardiomyocytes from both genotypes were therefore isolated and subjected to an hypoxia *in vitro* survival assay. Cardiomyocytes viability was determined either in normoxia (4h at 20% O₂) or in hypoxia (3h at 1% O₂ followed by 1h at 20% O₂). Contrasting with the *in vivo* results on myocardial infarct size, no difference in survival was observed between both groups either in normoxia or in hypoxia suggesting that the protective effects of ANGPTL4 upon infarction was not related to a direct protection on cardiomyocytes but rather related to an effect on the coronary vasculature during ischemia-reperfusion.

**Ischemia-reperfusion induced severe vascular alterations in *angptl4 ko* mice:** Transmission electron microscopy studies were then performed in order to compare the post-ischemic tissue injury in *angptl4*^{*LacZ*/*LacZ*} mice and *angptl4*^{*LacZ*/+} mice at the ultrastructural level. Reperfused cardiomyocytes exhibited no differences between both groups. Ultrastructural analysis showed large edematous areas, poor in inflammatory cells after 48h reperfusion in infarcted areas in *angptl4*^{*LacZ*/+}mice*,* whereas inflammatory cells had invaded the edematous region in *angptl4*^{*LacZ*/ *LacZ*} mice. Polynuclear neutrophils, macrophages, lymphocytes and fibrinogen deposits were observed. Whereas *angptl4*^{*LacZ*/+} mice showed normal pericytes coverage around endothelial cells, a large edematous space between endothelial cells and pericytes was observed in vessels from *angptl4*^{*LacZ*/*LacZ*} mice. Altogether, these data show increased vascular alterations that correlate with increased inflammatory infiltrate and infarct size in *angptl4*^{*LacZ*/*LacZ*} mice.

**Assessment of no-reflow areas and therapeutical potential of ANGPTL4:** As vascular alterations are associated with no-reflow, we further analyzed no-reflow in *angptl4*^{*LacZ*/ *LacZ*} and *angptl4*^{*LacZ*/+} mice. The anatomic zone of no-reflow was delineated by thioflavine S staining which binds the intact endothelium causing perfused tissue to fluoresce when exposed to ultraviolet light. The zone of no-reflow expressed as a percentage of the necrotic zone, was more important in the *angptl4*^{*LacZ*/*LacZ*} group (19± 1 %) compared to the *angptl4*^{*LacZ*/+} group (11± 2%) (p<0.05).

We next hypothesized that attenuation of vascular alterations by recombinant ANGPTL4 may lead to the enhancement of endothelial barrier function, which ultimately could protect mice from consequences ischemia-reperfusion in this model. Nevertheless, myocardial ischemia-reperfusion does not induce massive no-reflow in mice in these conditions, which renders it a difficult model to assess any potential therapeutic effect. As a proof of concept, we therefore sought to analyze the therapeutic potential of ANGPTL4 in a second non-rodent specie, *i.e.,* in an open-chest rabbit model of myocardial ischemia reperfusion in which the no-reflow phenomenon has been well established, as previously described (Hale, S.L., Mehra, A., Leeka, J. & Kloner, R.A. Postconditioning fails to improve no reflow or alter infarct size in an open-chest rabbit model of myocardial ischemia-reperfusion. Am J Physiol Heart Circ Physiol 294, H421-5 (2008)). Whereas the levels of circulating ANGPTL4 in ischemic pathologies has never been studied neither in man nor in any other species, a recent publication identify physiological determinants of plasma ANGPTL4 levels in humans, such as energy restriction and plasma FFAs and circulating levels of 20 to 100 ng/ml (Kersten, S. et al. Caloric restriction and exercise increase plasma ANGPTL4 levels in humans via elevated free fatty acids. Arterioscler Thromb Vasc Biol 29, 969-74 (2009)). We therefore performed I.V. injection of 100 ng/ml human recombinant ANGPTL4 in rabbits, 5 min. prior to ischemia-reperfusion. Infarct size expressed as a percentage of the risk zone (IS, % area at risk) was 57± 5% in the control group and 34± 7% in the ANGPTL4-treated group (Figure 1A); thus ANGPTL4 conferred cardioprotection. Then, the zone of no-reflow was studied. When expressed as a percentage of the area at risk, it was 41± 2% in the control group and 19± 6% in the ANGPTL4-treated group (Figure 1B). More importantly, when expressed as a percentage of the infarct size, it was 73± 4% in the control group and 55± 7% in the ANGPTL4-treated group (Figure 1C). Histological analyzes show that myocardial infarction consisted in a core of necrosis and huge hemorrhage within interstitial spaces in control group. In the ANGPTL4-treated group, the extent of hemorrhages, expressed as a percentage of total heart section area was decreased as compared to control group (5.7± 2% vs. 21.9± 6.4%, *p* < 0.05). Thus, these results therefore show that ANGPTL4 induces preservation of vascular network integrity as compared to controls and reduced the extent of no-reflow.

**Early post-ischemic control of vascular permeability is responsible for cardiac tissue protection** :As vasculoprotection was observed in ANGPTL4-treated rabbits, we tested the hypothesis that early post-ischemic vascular hyperpermeability might constitute a mechanism for increased vascular alterations, inflammation and myocardial damage observed in *angptl4*^{*LacZ*/*LacZ*} mice. We therefore analyzed vascular permeability after 45 min ischemia and only 4h of reperfusion in the heart of both *angptl4*^{LacZ/LacZ} and *angptl4*^{*LacZ*/+}mice. Vascular density was similar in both genotypes in basal conditions, and histological studies of HE slides and immunolabellings of CD31 and Mac3 after 45 min ischemia and 4h reperfusion showed that neither tissues necrosis nor vascular density or inflammation were different in both groups. Extravasation of Evans blue dye from the vascular cardiac beds and accumulating in the interstitium was then measured in *angptl4*^{LaCZ/LacZ} and in *angptl4*^{*LacZ*/+}mice. *Angptl4*^{LacZ/LacZ} mice displayed an increased cardiac vascular leakage compared to *angptl4*^{*LacZ*/+}mice 4h after ischemia (117.5± 15.2 *versus* 84.8± 2.7, p=0.032), whereas no significant difference was observed in basal conditions.

Then, in order to better characterize events responsible for increased permeability after 4h of reperfusion, we then performed injection of fluorescent microspheres that allowed localization of zones of vascular leakage thereby allowing us to conduct a detailed analysis of these areas by confocal microscopy. Microvessels and pericytes densities were similar between both groups as determined by CD31 and NG2 immunoreactivities. Since stability of VE-cadherin at adherens junctions is critical for maintenance of endothelial permeability and integrity, we therefore sought to investigate endothelial adherens junctions. An heterogenous pattern of VE-cadherin staining was observed in *angptl4*^{*LacZ*/+} mice; both intense and linear signals were adjacent to thinner signals. In contrast, in *angptl4*^{*LacZ*/*LacZ*} mice, ischemia-reperfusion injury induced more severe damages in endothelial junctions which were mainly disrupted as shown by a more discontinuous VE-Cadherin staining. In addition, these disrupted junctions massively allowed extravasation of FITC-beads. Comparatively, absence of FITC-beads in *angptl4*^{*LacZ*/+} mice indicated that the remaining stabilized junctions were sufficient to maintain barrier function in these mice.

In parallel, similar analyses were performed in basal conditions. No difference was observed in term of vascular density and pericytes coverage between both genotypes. We showed a strong VE-cadherin linear signal that labels a dense vascular network in *angptl4*^{*LacZ*/+} mice whereas this signal appeared also continuous but thinner in ***angptl4***^{*LacZ*/*LacZ*} mice. In accordance with data obtained in the modified Miles assay which did not reveal any difference of vascular leakage in basal conditions, FITC-labelled beads extravasation was absent in both groups. These observations therefore suggest that whereas VE-cadherin distribution appears less continuous and thinner in *angptl4*^{*LacZ*/*LacZ*} mice compared to control mice in basal conditions, this was not sufficient to increase basal vascular permeability in the heart. In contrast, ischemic conditions further induced junctional disassembly that eventually led to a higher vascular permeability in *angptl4*^{*LacZ*/*LacZ*} mice compared to control mice.

These results suggest that cardioprotection might be achieved through vasculoprotection and preservation of vascular endothelial cell barrier integrity. Altogether, we here describe ANGPTL4 as a new relevant physiological target during acute myocardial ischemia.

### Discussion:

Upon myocardial ischemia, hypoxia-inducible factor (HIF) proteins, the principal transcription factors involved in the regulation of transcriptional responses to hypoxia are rapidly activated, and induce VEGF-A expression that participates in regulating the angiogenic response but also causes vascular permeability and edema resulting in extensive injury to ischemic tissues. The first wave of VEGF-A release induces increase in endothelial cell permeability and tissue edema, contributing to ischemic-reperfusion injury, whereas the second peak might relate to the angiogenic reparative response. HIF also plays an essential role in triggering cellular protection and metabolic alterations from the consequences of oxygen deprivation. Here, we show that *angptl4* mRNA which has previously been shown to be induced by hypoxia in endothelial cells and in cardiomyocytes *in vitro* as well as in critical hind limb ischemia and stroke is also expressed in cardiac tissue from patients who died from an AMI. We further show that *angptl4* mRNA is also expressed in mouse in a model of AMI and we provide evidence that ANGPTL4 mediates post-ischemic tissue damage protection through preservation of vascular endothelial cell barrier integrity that limits no-reflow and the extent of AMI.

In pathological ischemic conditions, increased permeability which is predominantly controlled by endothelial junction stability is responsible for altered vascular integrity. Yang et al. showed that myocardial tissue levels of VE-cadherin were significantly decreased in the reflow and no-reflow myocardium compared to those in the non-ischemic myocardium, suggesting that microvascular structural integrity was damaged by ischemia/reperfusion (Yang, Y.J. et al. Post-infarction treatment with simvastatin reduces myocardial no-reflow by opening of the KATP channel. Eur J Heart Fail 9, 30-6 (2007)). In the *present in vivo* study, we show for the first time that *angptl4* ko mice display reduced adherens junctions stability and increased vascular permeability thereby leading to increased myocardial infarct size. VE-cadherin which constitutes the major component of the adherens junctions in endothelial cells, is required *in vivo* in the post-natal vasculature in order to maintain endothelial junction integrity and barrier function. VE-cadherin associates with VEGFR2 and regulates permeability. Indeed, VEGF-A stimulation induces dissociation of the VEGFR2/VE-cadherin complex, that can be prevented by blockade of Src, an essential molecule required for promoting the disruption of endothelial cell-cell contacts and paracellular permeability. We here showed that VE-cadherin distribution is disorganized in *angptl4* knockout mice, leading to destabilized adherens junctions, decreased vascular integrity and endothelial cell barrier function following cardiac ischemia-reperfusion. Other members of the angiopoietins family also display a role in the regulation of vascular permeability. In the mature vasculature, Angiopoietin-1 inhibits permeability induced by VEGF-A or mustard oil whereas Angiopoietin-2 causes inflammation *in vivo* by promoting vascular leakage. Interestingly, Angiopoietin-1 has been shown to phosphorylate Tie-2 and phosphatidylinositol 3-kinase, inducing activation of the GTPase Rac1, necessary to maintain cell-cell adhesion and to prevent hyperpermeability. Angiopoietin1 also promotes the activation of mDia through RhoA, resulting in the association of mDia with Src, thereby interfering with the ability of VEGF-A to initiate the activation of a Src-dependent intracellular signaling. Whether regulation of Src and/or Rac1 signaling pathways by ANGPTL4 may affect intracellular VE-cadherin distribution by stabilizing it at cell junctions through post-translational modification is beyond the scope of the present study but would deserve further investigation.

In addition, reperfused myocardial infarction is associated with cellular infiltration and acute inflammatory response. Cardiac repair after myocardial infarction is a dynamic and complex biological process that can be divided into the inflammatory phase, the proliferative phase, and the maturation phase. Whereas the inflammatory cascade is a prerequisite for healing of the infarcted myocardium, effective cardiac repair depends on mechanisms that also suppress the inflammatory response during vascular remodelling and that limit expansion of fibrosis to the noninfarcted myocardium. A critical point in the therapy against post-ischemia injury remains to contain deleterious persistent and expanding inflammatory response. Although numerous studies have focused on the expression and role of inflammatory mediators in the infracted myocardium, the cellular and molecular events responsible for downregulation and containment of the inflammatory cascade remain unknown. We here show that modulation of vascular permeability by ANGPTL4 might constitute a point of control that participates in decreasing the post-infarction inflammatory response and thus limit expansion of infarcted area.

We further show that early protection of vascular integrity by ANGPTL4 induces preservation of the microcirculatory network and lesser extent of hemorrhages that both participate in limiting the extent of no-reflow. This phenomenom is the result of yet uncompletely characterized anatomical changes of coronary microcirculation in which ANGPTL4 might play a crucial role through its vasculoprotective effect.

ANGPTL4 is also an inhibitor of LPL which is highly expressed in the heart and participates in lipoproteins hydrolysis as a source of fatty acids for uptake. Yu et al. have shown that mice that overexpress ANGPTL4 in the heart exhibit fasting hypertriglyridemia and develop left-ventricular dysfunction. This could mean that mice in which *angptl4* is deleted should have higher LPL activity which could participate in preventing cardiomyocyte death during infarction. Interestingly, if this event occurs *in vivo* in the ischemic heart, this effect is overcome by deleterious vascular effects observed in *angptl4* knockout mice that we describe here, rendering those mice more sensitive to myocardial ischemia/reperfusion injury. In addition, products generated from lipoprotein lipase-mediated hydrolysis of triglyceride-rich lipoproteins are reported to increase endothelial layer permeability through zonula occludens-1 (ZO-1) radial rearrangement and concurrent redistribution of F-actin from the cell body to the cell margins in human aortic endothelial cells. In addition, recent findings place VE-cadherin upstream of claudin-5, a key component of tight endothelial junction, in the maintenance of endothelial cell-cell junctions. Therefore, our results on the disorganization of endothelial adherens junctions in *angptl4* knockout mice suggest that ANGPTL4 could promote endothelial barrier function at multiple levels.

Finally, whereas cardiomyocytes were primarily recognized as a clinically therapeutic target of myocardial ischemia, few studies have focused on the importance of the cardiac vessels. More recently, clinical efforts are underway to block VEGF-A-mediated leak in patients after acute myocardial infarction or stroke. Such strategy may have a significant impact on reducing tissue injury and thereby minimizing the long-term consequences. Our findings suggest that ANGPTL4, by counteracting ischemia-induced disruption of endothelial cell junctions and subsequent increase in permeability observed in reperfused AMI might be promising for improved myocardial infarction therapy.

### EXAMPLE 2: PROTECTION AGAINST MYOCARDIAL INFARCTION AND NO-REFLOW THROUGH PRESERVATION OF VASCULAR INTEGRITY BY ANGIOPOIETIN-LIKE 4

### MATERIAL AND METHODS

The experiments were performed in accordance with the official regulations edicted by the French Ministry of Agriculture. This study conforms to the standards of INSERM (the French National Institute of Health) in accordance with European Union Council Directives (86/609/EEC).

**Myocardial ischemia-reperfusion experiments:** Ischemia-reperfusion protocol was performed on *angptl4*^{LacZ/+} and *angptl4*^{*LacZ*/*LacZ*} mice or rabbits using a standard technique described in Supplemental Materials. Rabbits randomly received either vehicle or human recombinant 55 kDa full-length ANGPTL4 (rhANGPTL4 10µg/kg i.v.).

**Modified Miles assay:** Male *angptl4*^{*LacZ*/*LacZ*} and *angptl4*^{Lac*Z*/+} mice were anesthetized using pentobarbital. For basal conditions, mice were injected into the tail vein with 1% Evans blue (200 µl) and sacrificed 4 h later. For the ischemia-reperfusion conditions, mice were subjected to coronary occlusion for 45 min and intravenously injected with 1% Evans blue (200 µl) prior the 4 h of reperfusion. At sacrifice, mice were perfused through the aorta with citrate buffer pH4. Blood, dye and buffer exited through an opening in the right atrium. Evans Blue was eluted for 18 h at 70°C in 1ml formamide. After centrifugation, absorbance at 620 nm was measured using a spectrophotometer. Extravasated Evans blue (ng) was determined from a standard curve and normalized to tissue weight (g).

Immunofluorescence study and confocal analyzis on cryosections: Immunofluorescence staining was performed as previously described (Brechot N, Gomez E, Bignon M, Khallou-Laschet J, Dussiot M, Cazes A, Alanio-Brechot C, Durand M, Philippe J, Silvestre JS, Van Rooijen N, Corvol P, Nicoletti A, Chazaud B, Germain S. Modulation of macrophage activation state protects tissue from necrosis during critical limb ischemia in thrombospondin-1-deficient mice. PLoS ONE. 2008;3:e3950.) and confocal analysis on cryosections is detailed in Supplemental Material.

**Immunoprecipitation and immunoblotting analyses:** For *in vivo* samples, mice subjected to 45 min ischemia-4 h reperfusion were anesthetized, injected into tail vein with 1mM Na₃VO₄ and 2mM H₂O₂ and dissected to remove left ventricle. For HUAECs experiments, 40000 cells/cm² were seeded in complete culture medium (Promocell) for 72 h. Cells were starved overnight and treated for 5 min with 100 ng/ml human recombinant VEGF₁₆₅ (Sigma) or with a mix containing 100 ng/ml (10 nM) VEGF and 5µg/ml (360 nM) human recombinant ANGPTL4 (Chomel C, Cazes A, Faye C, Bignon M, Gomez E, Ardidie-Robouant C, Barret A, Ricard-Blum S, Muller L, Germain S, Monnot C. Interaction of the coiled-coil domain with glycosaminoglycans protects angiopoietin-like 4 from proteolysis and regulates its antiangiogenic activity. Faseb J. 2009;23:940-949.) before to be washed twice with Ca/MgPBS. Proteins were extracted, immunoprecipitated for VEGFR2 and analyzed by Western blotting as described in Supplementary Material.

**Isolation of cardiomyocytes and viability assay:** Cardiac myocytes were isolated as described in Supplemental Material and incubated in an anaerobic chamber containing a humidified atmosphere of 5% CO₂ and 95% N₂ for 3 h. Experimental medium was changed to serum-free, glucose-free. Cardiac myocyte survival was measured by staining cells with trypan blue (Sigma).

**Statistical analyses:** Mann-Whitney or Student's tests were used to assess the statistical differences between groups or conditions (GraphPad Prism 4, GraphPad Software). Error bars indicate SEM and *, P < 0.05; **, P < 0.001; ***, P < 0.0001.

### SUPPLEMENTAL MATERIALS

### Expended Methods and Results

**Animals and genotyping:** Genotype was determined by PCR of tail genomic DNA as previously described (Gomez, 2010, submitted). Eight to 12 weeks of age *angptl4*^{LacZ/+} and *angptl4*^{*LacZ*/*LacZ*} knock-out male mice, intercrossed in C57/B16 mice for more than 8 generations, were subjected to myocardial infarction protocols or used as control in basal conditions.

**Mice myocardial ischemia-reperfusion experiments:** Mice were anesthetized by an intraperitoneal injection of sodium pentobarbital. Myocardial infarction with occlusion of the left coronary artery was performed for 45 mn and tissues were reperfused for 1 h to 3 weeks. For *angptl4* expression study WT mice underwent 1 h, 3 h, 24 h, 48 h, 72 h, 1 week, 2 weeks, 3 weeks of reperfusion. To assess infarct size and for immunohistochemistry (IHC) or ultrastructural studies, male *angptl4*^{Lac*Z*/+} and *angptl4*^{*LacZ*/*LacZ*} mice were reperfused during either 4 h or 48 h after ischemia. The area at risk was identified by Evans blue staining at 48 h after ischemia, and the infarct area was identified by 2,3,5-triphenyltetrazolium chloride (TTC) staining. The area at risk was identified as the non blue region and expressed as a percentage of the left ventricle weight. The infarcted area was identified as the TTC-negative zone and expressed as a percentage of the area at risk. To measure no-reflow, the chest was reopened and thioflavine S (4%; 1.5 ml/kg) was infused through the left atrium four hours after the onset of reperfusion. The hearts were then perfused retrogradely with Alcian blue (0.5%) and cut into slices. Slices were photographed using UV light to identify the region of no-reflow. The areas of infarct and risk zone were determined as defined above. Ultrastructural analyses were performed on a Hitachi H-9500 electron microscope.

Immunofluoresence study and confocal analyzis on cryosections: Mice subjected to ischemia and 4 h of reperfusion were anesthetized with ketamine and xylazine injected intraperitonally. FITC-beads (20 µl) were injected into the femoral vein as previously described ⁴⁴. The chest was opened rapidly, and the vasculature was perfused for 2 min at a pressure of 120 mmHg with 1% paraformaldehyde. The heart was then placed into 1% paraformaldehyde for 1 h at room temperature, rinsed with PBS and frozen for cryostat sectioning. Endothelial cells, pericytes and adherens junctions were identified with rat anti-CD31 (BD Pharmingen), rabbit anti-NG2 (Chemicon) and rat anti-VE-Cadherin (personal gift from E. Dejana, IFOM) antibodies, respectively. Confocal sections were imaged on a Leica SP5 microscope (Leica Microsystems GmbH) using a 63x (NA = 1.4) oil objective. An increment of 0.117 µm between each section was used. 3D reconstruction of the different structures was obtained using the LABELVOXEL and the SURFACEGEN modules in Amira 5.2.1 software (Visage Imaging GmbH).

**Immunoprecipitation and immunoblotting analyses :** Proteins were extracted on ice in 20 mM Tris-HCl, pH 7.6, 150 mM NaCl, 0.1% DOC, 0.5% NP-40, 10% glycérol, 1 mM β-glycerophosphate, 1 mM NaF, 2.5mM Na pyrophosphate, 1 mM Na₃VO₄ and a cocktail of protease inhibitors (Calbiochem). Lysates were split for immunoprecipitation and for total extracts immunoblottings. For immunoprecipitation, extracts were precleared for 60 min with protein A-agarose beads, incubated overnight with anti-VEGFR-2 (Cell signaling), and the immunocomplexes were collected on protein A-agarose beads for 3 h. Proteins were eluted by boiling for 10 min in reducing laemmli sample buffer. Samples were analyzed by SDS-PAGE followed by Western blotting on nitrocellulose membrane. Anti-VEGFR2 (Cell signaling), anti-VE-Cadherin (Santa Cruz), anti-Src kinase family (Cell signaling), anti-phospho Src family Tyr- 416 (Cell signaling) antibodies were used. Signal was revealed by Attophos chemiluminescence (Promega) and band intensity was quantified by Quantity One 1-D Analysis Software (Biorad).

**Isolation of cardiomyocytes and viability assay:** Under anesthesia, the heart was removed from the chest and was cannulated. The heart was perfused for 4 min with tyrode buffer ([mM] NaCl 113 ; KCl 4.7 ; KH₂PO₄ 0.6; Na₂HPO₄ 0.6 ; HEPES 10 ; MgSO₄ 1.2 ; NaHCO₃ 12 ; KHCO₃ 10 ; taurine 30 ; phenol red 0.032 ; glucose 5.5 ; with pH ajdusted to 7.46 with NaOH 1N) at constant pressure and 37°C. Perfusion was switched to an enzyme solution ([mM] NaCl 113; KCl 4.7 ; KH₂PO₄ 0.6 ; Na₂HPO₄ 0.6 ; HEPES 10 ; MgSO₄ 1.2 ; NaHCO₃ 12 ; KHCO₃ 10 ; taurine 30 ; phenol red 0.032 ; glucose 5.5 ; CaCl₂ 0.0125 ; with pH adjusted to 7.46 with NaOH 1N) containing 0.1 mg/ml liberase blendzyme IV, (Roche diagnostics) and 0.14 mg/ml trypsin (Sigma). When hearts became swollen and turn slightly pale, the atria and aorta were removed; the left ventricle were cut into small pieces and gently triturated. Cell suspension was transferred into a stopping buffer ([mM] NaCl 113 ; KCl 4.7 ; KH₂PO₄ 0.6 ; Na₂HPO₄ 0.6 ; HEPES 10 ; MgSO₄ 1.2 ; NaHCO₃ 12 ; KHCO₃ 10 ; taurine 30 ; phenol red 0.032 ; glucose 5.5 ; CaCl₂ 0.0125 ; calf serum 5% ; with pH adjusted to 7.46 with NaOH 1N). Extracellular calcium was added incrementally up to 1.0 mM. All cells studied were rod-shaped, had clear cross-striations and lacked any visible vesicles on their surfaces.

**Rabbit experiments:** New Zealand rabbits (2.5-3.0 kg) were anesthetized using zolazepam, tiletamine and pentobarbital (all 20-30 mg/kg i.v.). The animals were intubated, mechanically ventilated and a left thoracotomy was performed. A suture was passed beneath a major branch of the left coronary artery through a short propylene tubing to form a snare. Rabbits then randomly received either vehicle or human recombinant 55 kDa full-length ANGPTL4 ¹² (10 µg/kg i.v.). Five minutes after, coronary artery occlusion (CAO) was induced during 30-min by pulling the snare through the tubing. Reperfusion was subsequently induced by releasing the snare. The chest was then closed in layers. Four hours after the onset of reperfusion, the chest was reopened and thioflavine S (4%; 1.5 ml/kg) was infused through the left atrium. Rabbits were then sacrificed using pentobarbital followed by potassium chloride. After excision, the hearts were perfused retrogradely with Alcian blue (0.5%) and cut into slices. Slices were photographed using UV light to identify the region of no-reflow. The areas of infarct and risk zone were determined as in mice.

**Ultrasound analysis of cardiac parameters:** Mice were subjected to ultrasound measurements using an echocardiograph (Vivid 7, GE Medical Systems Ultrasound) equipped with a 12-MHz linear transducer.

**Real-time quantitative PCR analysis (RT-qPCR) :** Mice subjected to ischemia and 4 h or 18 h reperfusion were anesthetized, injected into tail vein and intracardiacly with 1mM Na₃VO₄ and 2mM H2O2 and dissected to remove left ventricle. Total RNA was isolated by extraction with TRIzol (Invitrogen). Reverse transcription, quantitative PCR (in triplicate) and analysis were performed as previously described (Xu Y, Yuan L, Mak J, Pardanaud L, Caunt M, Kasman I, Larrivee B, Del Toro R, Suchting S, Medvinsky A, Silva J, Yang J, Thomas JL, Koch AW, Alitalo K, Eichmann A, Bagri A. Neuropilin-2 mediates VEGF-C-induced lymphatic sprouting together with VEGFR3. J Cell Biol. 2010;188:115-130.). mRNA expression level was normalized to the housekeeping gene encoding glyceraldehyde-3-phosphate dehydrogenase (GAPDH). Fold changes were calculated using the comparative Ct method.

***In situ* hybridization (ISH) and immunohistochemistry (IHC) analyses:** Paraffin blocks of human myocardial infarcts were obtained from the Pathology Department of Georges Pompidou European Hospital, Paris, France. The presence of infarcted areas was assessed on standard HE staining and adjacent slides were used for ISH and IHC analyses. ISH using human or mouse *angptl4* probes and IHC immunolabellings anti-CD45, -Mac3, and-CD31 were performed as previously described (Brechot N, Gomez E, Bignon M, Khallou-Laschet J, Dussiot M, Cazes A, Alanio-Brechot C, Durand M, Philippe J, Silvestre JS, Van Rooijen N, Corvol P, Nicoletti A, Chazaud B, Germain S. Modulation of macrophage activation state protects tissue from necrosis during critical limb ischemia in thrombospondin-1-deficient mice. PLoS ONE. 2008;3:e3950.).

### RESULTS

### Early post-ischemic vascular integrity is altered in angptl4^{LacZ/LacZ} mice

*angptl4*^{*LacZ*/*LacZ*} mice in which the *angptl4* locus was replaced by a *lacZ* reporter gene were generated. We first analyzed vascular permeability after 45 min ischemia and 4 h reperfusion in the heart of both *angptl4^{LacZlLacZ}* and *angptl4*^{*LacZ*/+} mice. Histological analyzes showed that tissue damage was equivalent in both groups at this early time point. *Angptl4*^{*LacZ*/*LacZ*} mice displayed an increased vascular leakage compared to *angptl4*^{*LacZ*/*+*}mice 4h after ischemia (117.5±15.2 *versus* 84.8±2.7 µg/ml, p<0.05), whereas no significant difference was observed in basal conditions. Importantly, *angptl4*^{*LacZ*/*Lacz*} mice did not exhibit any functional cardiac defects or abnormal vascular morphology both in basal or in ischemic conditions.

Then, fluorescent microspheres were injected thus allowing the localization of areas of vascular leakage. In basal conditions, FITC-beads did not extravasate in both groups. After 45 min ischemia- 4 h reperfusion, no FITC-bead was observed in *angptl4*^{*LacZ*/+} mice whereas extravasation of fluoresecent microspheres was detected in *angptl4*^{*LacZ*/*LacZ*} mice indicating that endothelial integrity was altered. Since stability of adherens junctions is critical for maintenance of endothelial permeability and integrity, we sought to investigate VE-Cadherin distribution at endothelial adherens junctions in basal conditions and in ischemia-reperfusion model. In non-ischemic myocardium from both genotypes, a VE-Cadherin linear signal labelling a dense vascular network was observed. In contrast, after ischemia-reperfusion, a heterogeneous pattern of VE-Cadherin staining was observed in *angptl4*^{*Lacz*/+} mice; both intense and linear signals were adjacent to thinner signals. In *angptl4*^{*LacZ*/*LacZ*} mice, ischemia-reperfusion injury induced more severe damages in endothelial junctions, which were mainly disrupted as shown by a more systematic discontinuous VE-Cadherin staining. 3D-images rebuilt from confocal pictures of *angptl4*^{*LacZ*/*LacZ*} heart sections stained with anti-CD31 and anti-NG2 antibodies further confirmed extravasated FITC-beads from blood vessels.

These observations suggest that coronary vascular integrity is fragile and junction disassembly is more frequent in *angptl4*^{*LacZ*/*LacZ*} mice during ischemic conditions, thereby leading to increased vascular permeability.

### Post-ischemic decrease in VEGFR2 and VE-Cadherin expression combined to increase Src kinase phosphorylation downstream the VEGFR2 in angptl4^{LacZ/LacZ} mice

In the vasculature, VEGFR2 and VE-Cadherin form complexes that are transiently dissociated upon VEGF binding to VEGF-R2. During myocardial infarction, ischemia promotes VEGF expression that leads to vascular permeability and edema. We therefore investigated whether enhanced VEGFR2/VE-Cadherin complex disassembly might constitute the mechanism responsible for an increased junctional disruption in *angptl4*^{*LacZ*/*LacZ*} mice after ischemia-reperfusion injury.

Using RT-qPCR, *vegfr2* and *ve-cadherin* mRNA expression were quantified in the left ventricle in *angptl4^{LacZlLacZ}* and *angptl4*^{*LacZ*/+} mice, in control conditions or after 4 h or 18 h reperfusion. *ve-cadherin* and *vegfr2* mRNA expression was similar in both groups in basal conditions. After 4 h reperfusion, massive decrease in *vegfr2* and *ve-cadherin* mRNA expression was observed in *angptl4^{LacZlLacZ}* mice compared to control mice (58±3 *versus* 34±3 % for *ve-cadherin* and 70±2 *versus* 7±5 % for *vegfr2,* p<0.001). This down-regulation was maintained after 18 h reperfusion for the *vegfr2* mRNA (decrease of 66±2% for *angptl4^{LacZlLacZ}* mice, p<0.001). Protein levels were also affected as shown by western blot analyzes performed using total extracts from left ventricles in control and ischemic conditions.

Src kinase is required in VEGF-mediated permeability through its role in dissociating the VEGFR2/VE-Cadherin complex. To further determine the mechanism leading to early post-ischemic junctions alteration in *angptl4^{LacZlLacZ}* mice, Src kinase signaling downstream of VEGFR2 was analyzed in control conditions and after ischemia-reperfusion. Left ventricles lysates were immunoprecipitated for VEGFR2 followed by immunoblotting for VEGFR2, VE-Cadherin, Src and phospho-Src. In basal conditions, VEGFR2/VE-Cadherin formed complexes in both genotypes. A transient destabilization of VEGFR2/VE-Cadherin complexes was observed at 4 h reperfusion and was restored after 18 h reperfusion in *angptl4*^{*LacZ*/+} mice whereas VE-Cadherin remains dissociated from VEGFR2 in *angptl4*^{*LacZ*/*LacZ*} mice. In addition, immunoblottings showed an increased Src kinase recruitment and phosphorylation after 4 h and 18 h of reperfusion in *angptl4*^{*LacZ*/*LacZ*} compared to *angptl4*^{*LacZ*/+} mice.

These results show that a decrease in *vegfr2* and *ve-cadherin* expression combined to an increase in Src kinase phosphorylation downstream the VEGFR2 lead to VEGFR2/VE-Cadherin complex dissociation responsible for massive disorganisation of VE-Cadherin in endothelial adherens junctions in *angptl4*^{*LacZ*/*LacZ*} mice following ischemia-reperfusion.

### Infarct size, no-reflow and post-ischemic inflammation are increased in angptl4^{LacZ/LacZ} mice

We next hypothesized that alteration of vascular integrity in *angptl4*^{*LacZ*/*LacZ*} mice might translate to abnormal myocardial reperfusion and major heart tissue damage at 48 h reperfusion. Indeed, infarct size was increased in *angptl4*^{*LacZ*/*LacZ*} mice compared to *angptl4*^{*LacZ*/+} mice (47±3 *versus* 36± 3%, p<0.01). In addition, the no-reflow was more important in the *angptl4*^{*LacZ*/*LacZ*} group compared to the *angptl4*^{*LacZ*/+} mice, when expressed as a percentage of the necrotic zone (19±1 *versus* 11±2 %, p<0.05).

Necrosis, hemorrhages and edema were also quantified (score 1 to 3) on HE-stained sections from infarcted hearts of both genotypes. In accordance with increased infarct size, tissue necrosis was largely increased in *angptl4*^{*LacZ*/*LacZ*} compared to control mice (2.5±0.6 *versus* 1.2±0.2). Assessment of hemorrhages and edema revealed a more severe tissue injury in *angptl4*^{*LacZ*/*LacZ*} (2.3±0.6 *versus* 1*.*1±0.2 and 2.3±0.2 *versus* 0.8±0.2, respectively). The post-ischemic inflammatory response was also analyzed in both genotypes. Macrophage density was significantly higher in infarcted areas in *angptl4*^{*LacZ*/}*^{LacZ} versus angptl4*^{*LacZ*/+} mice, whereas no statistical difference was observed between both groups in control non-infarcted areas.

We then analyzed vascular density in the core infarct area and in the periphery using CD31 staining. A similar diminished microcapillary density was quantified in both genotypes in the central infarcted areas as compared to the periphery. No difference was quantified between both genotypes in both areas.

Transmission electron microscopy study was further performed in order to assess tissue injury at the ultrastructural level. Analysis of reperfused infarcted areas did not show cardiomyocyte structural differences between both groups (see « C₁ to ₄ » in Figure IVA and IVB) but large edematous areas, poor in inflammatory cells were observed in *angptl4*^{*LacZ*/+} mice, whereas inflammatory cells had already invaded the edematous region in *angptl4*^{*LacZ*/*LacZ*} mice. Polynuclear neutrophils, macrophages, lymphocytes and fibrinogen deposits were only observed in *angptl4*^{*LacZ*/*LacZ*} mice. Altogether, these data indicate increased vascular alterations that correlate with increased inflammatory infiltrate in *angptl4*^{*LacZ*/*LacZ*} mice.

As hypoxic activation of *angptl4* mRNA has been reported in cardiomyocytes, likely mediated by hypoxia-inducible factor 1, we also hypothesized ANGPTL4 might also affect cardiomyocytes survival. Indeed, LacZ staining performed on whole-mount, HE-stained and CD31-immunostained sections from *angptl4*^{*LacZ*/*LacZ*} mice also revealed that both cardiomyocytes and ECs express *angptl4* after ischemia-reperfusion injury. *In situ* hybridization (ISH) further showed *angptl4* mRNA expression was induced as soon as 3 h following ischemia and up to 2 weeks after reperfusion, whereas *angptl4* mRNA was not expressed in the non-ischemic area. ISH in cardiac samples from patients who died from AMI also revealed *angptl4* mRNA expression in cardiomyocytes and in ECs. Cardiomyocytes from both genotypes were therefore isolated and subjected to an *in vitro* survival assay. No difference in cardiomyocytes survival was observed *in vitro* between both groups either in normoxia or in hypoxia suggesting that ANGPTL4 does not have a direct effect on cardiomyocytes.

### Recombinant ANGPTL4 stabilizes VEGFR2/VE-Cadherin complex in response to VEGF

We next investigated whether rhANGPTL4 could confer protection of VEGFR2/VE-Cadherin complexes disassembly in ECs. Confluent HUAECs were stimulated for 5 min with VEGF alone or with rhANGPTL4. Cell lysates were immunoprecipitated for VEGFR2 followed by immunoblotting for VEGFR2, VE-Cadherin, Src and phospho-Src. The pre-existing VEGFR2/VE-Cadherin observed in control conditions is rapidly disrupted within 5mn VEGF stimulation. This complex was protected from dissociation in cells treated with both VEGF and rhANGPTL4 (. Src kinase and phospho-Src immunoblottings revealed that VEGF-mediated VEGFR2/VE-Cadherin destabilization was correlated with an increased Src phosphorylation downstream VEGFR2 which was partially blocked in cells treated with both VEGF and rhANGPTL4.

These *in vitro* experiments provide evidence that rhANGPTL4 protects from VEGF-induced VEGFR2/VE-Cadherin complex destabilisation through inhibition of Src kinase signaling.

### Assessment of therapeutic cardioprotective effect of rhANGPTL4

We next hypothesized that attenuation of vascular alterations by rhANGPTL4 may lead to the enhancement of endothelial barrier function, which ultimately could protect from ischemia-reperfusion. As myocardial ischemia-reperfusion does not induce massive no-reflow in mice in these conditions, we therefore sought to analyze the therapeutic potential of ANGPTL4 in a non-rodent specie, *i.e.,* in an open-chest rabbit model of myocardial ischemia-reperfusion in which the no-reflow phenomenon has been well established.

We therefore performed intravenous injection of 10 mg/kg rhANGPTL4, 5 min. prior to ischemia-reperfusion. Infarct size expressed as a percentage of the risk zone (IS, % area at risk) was 57±5% in the control group and 34±7% in the rhANGPTL4-treated group (p<0.01). Then, the zone of no-reflow was studied. When expressed as a percentage of the area at risk, it was 41±2% in the control group and 19±6% in the rhANGPTL4-treated group (p<0.05). More importantly, when expressed as a percentage of the infarct size, it was 73±4% in the control group and 55±7% in the rhANGPTL4-treated group (p<0.05). Histological analysis showed that myocardial infarction consisted in a core of necrosis and huge hemorrhage within interstitial spaces in control group. In the rhANGPTL4-treated group, the extent of hemorrhages was decreased (5.7±2% *versus* 21.9±6.4%, expressed as a percentage of total heart section area, p<0.05).

Thus, these results show that rhANGPTL4 induces preservation of vascular integrity that reduces infarct size, hemorrhage and no-reflow and thus confers cardioprotection.

### DISCUSSION

Upon AMI, hypoxia-inducible factor (HIF) proteins, the major transcription factors involved in the regulation of responses to hypoxia are rapidly activated, and induce VEGF-A expression that participates in regulating the angiogenic response but also causes vascular permeability and edema resulting in extensive injury. We showed here that *angptl4* mRNA which has previously been shown to be induced by hypoxia in ECs and in cardiomyocytes in *vitro* as well as in critical hind limb ischemia and stroke is also expressed in cardiac tissue from patients who died from AMI. We further provided evidence that ANGPTL4 mediates post-ischemic damage protection through preservation of vascular endothelial cell barrier integrity that limits no-reflow and the extent of AMI.

In pathological ischemic conditions, increased permeability, which is controlled by endothelial junction stability, is responsible for altered vascular integrity. VE-cadherin which constitutes the major component of the adherens junctions between ECs, is required *in vivo* in the post-natal vasculature in order to maintain endothelium integrity and barrier function. It was showed that myocardial VE-Cadherin is significantly decreased in the ischemic myocardium, suggesting that microvascular integrity is damaged by ischemia/reperfusion. VE-cadherin associates with VEGFR2 and regulates permeability. Indeed, systemic VEGF-A injection, thereby activating VEGFR2, induces dissociation of the VEGFR2/VE-Cadherin complex. Here we showed durable dissociation of VEGFR2/VE-Cadherin complexes and altered VE-cadherin distribution in *angptl4* knockout mice, causing disrupted adherens junctions and decreased ECs barrier function following AMI. Gene expression analysis revealed i) a more prominent diminished *ve-cadherin* mRNA levels, ii) a prolonged decrease of *vegfr2* mRNA levels in *angptl4* knockout mice subjected to AMI. Decreased levels of *vecadherin* and *vegfr2* gene expression, and therefore of VEGFR2/VE-Cadherin complexes in response to ischemia might participate to junctions disruptions and altered endothelial integrity following AMI in *angptl4*^{*LacZ*/*LacZ*} mice.

Src is an essential molecule required for promoting the disruption of ECs contacts and paracellular permeability. We here provide evidence for an enhanced i) Src kinase recruitment at the VEGFR2/VE-Cadherin complex and ii) Src kinase phosphorylation, leading to a more severe destabilization of the VEGFR2/VE-Cadherin complex in *angptl4*^{*LacZ*/*LacZ*} mice subjected to AMI. Other members of the angiopoietin family also display a role in the regulation of vascular permeability. Angiopoietin-1 phosphorylates Tie-2 and phosphatidylinositol 3-kinase, inducing activation of the GTPase Rac1, necessary to maintain cell-cell adhesion and also activates mDia, resulting in the sequestration Src. Whether regulation of Src/mDia or Rac1 signaling pathways by ANGPTL4 may affect intracellular VE-cadherin distribution by stabilizing it at cell junctions through post-translational modification would deserve further investigation.

In addition, reperfused myocardial infarction is associated with cellular infiltration and acute inflammatory response. A critical point in post-ischemic therapy remains to contain deleterious persistent and expanding inflammatory response. We here show that altered vascular integrity in *angptl4*^{*LacZ*/*LacZ*} mice might suppress a point of control that participates to limiting both the post-infarction inflammatory response and the expansion of infarcted area. We also showed that recombinant ANGPTL4 induces both preservation of the microcirculatory network and lesser extent of hemorrhages that both participate in limiting the extent of no-reflow. This phenomenon is the result of yet incompletely characterized anatomical changes of coronary microcirculation (Kloner RA, Ganote CE, Jennings RB. The "no-reflow" phenomenon after temporary coronary occlusion in the dog. J Clin Invest. 1974;54:1496-1508.) in which ANGPTL4 might play a crucial role through its vasculoprotective effect. In addition, recent findings place VE-cadherin upstream of claudin-5, a key component of tight endothelial junction, in the maintenance of endothelial cell-cell junctions. Therefore, our results on the disorganization of endothelial adherens junctions in *angptl4*^{*LacZ*/*LacZ*} mice suggest that ANGPTL4 could promote endothelial barrier function at multiple levels.

Finally, whereas cardiomyocytes were primarily recognized as a clinically therapeutic target of myocardial ischemia, few studies have focused on the importance of the cardiac vessels. Our findings show that ANGPTL4 counteracts increase in permeability observed in reperfused AMI. Clinical efforts are also underway to block VEGF-A-mediated leak in patients after acute myocardial infarction or stroke. The search for combined strategies will certainly have a significant impact on reducing tissue injury, improving coronary microcirculation and thereby improving myocardial infarction therapy.

### REFERENCES

Throughout this application, various references describe the state of the art to which this invention pertains.

## Claims

1. An ANGPTL4 polypeptide for use in the prevention of no-reflow during the treatment of a coronary heart disease.

2. The ANGPTL4 polypeptide for use according to claim 1 wherein said coronary heart disease is myocardial infarction or ST-segment elevation myocardial infarction.

## Patentansprüche

1. ANGPTL4-Polypeptid für die Verwendung bei der Prävention des No-Reflow-Phänomens bei der Behandlung einer koronaren Herzerkrankung.

2. ANGPTL4-Polypeptid für die Verwendung nach Anspruch 1, wobei die genannte koronare Herzerkrankung ein Myokardinfarkt oder ein ST-Hebungsinfarkt ist.

## Revendications

1. Polypeptide d'ANGPTL4 pour utilisation dans la prévention de la non reperfusion au cours du traitement d'une maladie coronarienne.

2. Polypeptide d'ANGPTL4 pour usage selon la revendication 1, dans lequel ladite maladie coronarienne est un infarctus du myocarde ou un infarctus du myocarde avec élévation du segment ST.
